# EUROPEAN PATENT SPECIFICATION

(11) **EP 2 841 154 B1**
(45) Date of publication and mention of the grant of the patent: **08.06.2022**
(21) Application number: 13781035.4
(22) Date of filing: 19.04.2013
(51) Int. Cl.: A61B 18/14, A61N 1/36

(54) **BLADDER TISSUE MODIFICATION FOR OVERACTIVE BLADDER DISORDERS**
BLASENGEWEBEMODIFIZIERUNG FÜR HYPERAKTIVE BLASENSTÖRUNGEN
MODIFICATION DU TISSU DE LA VESSIE EN CAS DE TROUBLES ASSOCIÉS À UNE VESSIE HYPERACTIVE

(30) Priority: 22.04.2012 US 201261636686 P; 20.05.2012 US 201261649334 P
(43) Date of publication of application: 04.03.2015
(60) Divisional application: 22167023.5
(73) Proprietor: NewUro, B.V., 1217 KR Hilversum (NL)
(72) Inventor: Ben-Ezra, Omry, 63113 Tel Aviv (IL); Avneri, Itzhak, 64233 Tel Aviv (IL)
(74) Representative: Clark, Jane Anne
(86) International application number: PCT/IB2013/001203
(87) International publication number: WO 2013/160772

(56) References cited:
- WO-A1-2005/067791
- WO-A1-2013/016590
- WO-A1-2013/052848
- WO-A1-2013/052852
- US-A1- 2004 186 468
- US-A1- 2004 186 468
- US-A1- 2007 282 184
- US-A1- 2011 301 662
- US-A1- 2011 301 662
- None

## Description

### BACKGROUND OF THE INVENTION

### 1. Background.

The present invention relates to medical devices .

More specifically, the present invention relates to systems, devices, and techniques for treating conditions of hollow organs in general. The relief of symptoms caused by overactive urinary bladder is discussed in particular.

Tissue ablation is a known technique for the treatment of various bodily disorders. Currently, ablation is used to eliminate pathological tissue (such as ablation of tumors or skin lesions), to remodel physical structures of tissue (such as in ablation of hypertrophied prostate to alleviate obstruction of urine, or ablation of pharyngeal tissue to alleviate snoring), to eliminate hyperactive normal tissue (renal nerve denervation to reduce blood pressure, uterine ablation to reduce menstrual bleeding), and to modify the electrical conductivity of tissue (such as in treating cardiac arrhythmia). Tissue ablation is often used to treat cardiac rhythm disorders in particular, especially atrial fibrillation. The methods and devices for performing such procedures in a beating heart are documented and described in the art. Many ablation procedures, however, are lengthy, demand visualization, imaging and/or localization, and are typically performed in specialized labs at significant costs. While using ablation of cardiac tissue to modify tissue conductivity within an organ in order to relieve arrhythmia has been known for years, this treatment modality is applied to cardiac tissue out of the belief that only excitable and conductive tissue, such as cardiac tissue can be used in this way.

Overactive bladder is typically caused by urinary muscle spasms that cause an urgency, often an unstoppable urgency, to urinate. Overactive bladder is common in older adults and is estimated to affect more than one in ten adults in the United States. Current treatments for overactive bladder include bladder training, pelvic floor exercises, and anticholinergics or similar drugs for more difficult cases. Anticholinergics can block the nerve signals related to bladder muscle contraction and can even increase bladder capacity. The use of anticholinergics, however, can result in many side effects such as dry mouth, constipation, blurred vision, and increased heart beat. Therefore, anticholinergics are not often recommended for patients with glaucoma, urinary retention, or gastrointestinal diseases. Other drug classes may be applied to relax bladder muscles but are often associated with undesirable side effects as well. In extreme cases, surgical procedures are used. These surgical procedures include bladder augmentation, the surgical enlargement of the bladder by addition of intestinal tissue to the bladder tissue, and the implantation of a bladder pacemaker. Such surgical procedures, however, are highly invasive and can involve the permanent implantation of a device which can lead to many related complications.

Thus, improved devices and methods for treatment of urinary disorders are desired. These improved device and methods may be specifically designed to treat symptoms and disorders, including overactive urinary bladder, not traditionally treated with ablation or similar procedures, desirably without the side effects and complications that commonly occur with the use of current drugs and devices.

### 2. References of Interest.

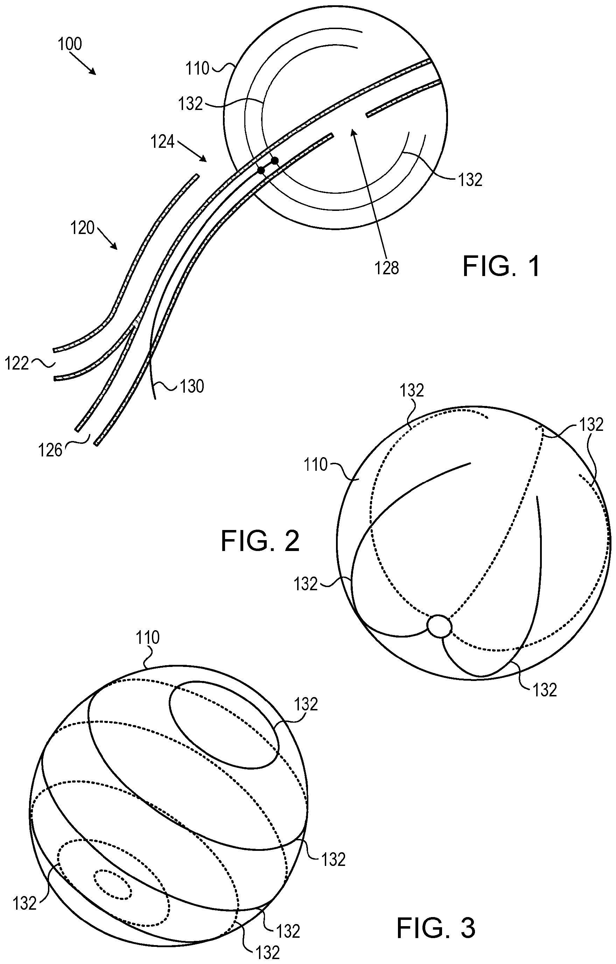

The following U.S. Patents, U.S. Patent Publications, and PCT Publications may be of interest: U.S. Patent Nos. 8,137,342, 8,007,496, 7,892,229, 7,850,681, 7,846,153, 7,837,720, 7,813,313, 7,744,594, 7,761,169, 7,655,006, 7,655,005, 7,632,268, 7,648,497, 7,625,368, 7,556,628, 7,527,622, 7,507,234, , 7,500,973, 7,410,486, 7,381,208, 7,371,231, 7,326,235, 7,357,796, 7,300,433, 7,288,089, 7,288,087, 7,278,994, 7,278,991, 7,220,257, 7,195,625, 7,192,438, 7,101,387, 7,101,368, 7,083,614, 7,081,112, 7,074,233, 7,060,062, 7,022,120, 7,001,378, 6,997,924, 6,875,209, 6,740,108, 6,692,490, 6,629,535, RE038229, 6,496,737, 6,458,098, 6,353,751, 6,283,989, 6,223,085, 6,161,049, 6,097,985, 6,083,255, 6,053,937, 6,024,743, 6,001,093, 5,992,419, 5,989,284, 5,902,251, 5,827,273, 5,800,486. 5,649,973, 5,599,294, 5,578,008, 5,509,929, 5,480,417, 5,470,352, 5,405,346, 5,380,319, 5,188,602, 5,150,717, 5,106,360, 5,057,106, 5,056,531, 4,808,164; U.S. Pub. Nos. 2013/0066308, 2013/0018281, 2012/0143179, 2012/0130363, 2012/0116384, 2012/0101490, 2012/0071873, 2012/0071870, 2012/0065636, 2012/0059368, 2012/0029500, 2012/0022520, 2012/0016358, 2012/0004656, 2012/0004654, 2011/0319880, 2011/0306904, 2011/0301662, 2011/0264086, 2011/0264085, 2011/0257647, 2011/0166570, 2011/0152855, 2011/0152839, 2011/0118719, 2011/0112432, 2011/0098694, 2011/0034976, 2011/0028886, 2011/0082450, 2010/0114087, 2010/0305562, 2010/0030204, 2010/0286753, 2010/0286688, 2010/0280510, 2010/0234840, 2010/0198066, 2010/0179530, 2010/0168734, 2010/0160906, 2010/0114087, 2010/0076425, 2010/0076402, 2010/0049192, 2010/0049186, 2010/0049182, 2010/0049031, 2010/0004650, 2009/0318914, 2009/0306644, 2009/0281532, 2009/0248012, 2009/0163906, 2009/0131928, 2009/0076494, 2009/0018533, 2008/0312642, 2008/0262489, 2008/0249518, 2008/0223380, 2008/0172050, 2008/0140070, 2008/0140067, 2008/0125765, 2008/0097427, 2008/0077174, 2008/0004613, 2007/0293854, 2007/0282184, 2007/0129725, 2007/0088247, 2007/0078451, 2007/0066973, 2007/0049918, 2007/0038203, 2007/0005050, 2006/0259029, 2006/0253178, 2006/0253113, 2006/0167442, 2006/0118127, 2006/0009758, 2005/0251125, 2005/0228370, 2005/0165389, 2005/0131500, 2005/0124843, 2005/0107783, 2005/0096647, 2004/0243199, 2004/0186468, 2004/0172112, 2004/0147915, 2004/0133254, 2003/0069619, 2003/0060813, 2003/0060762, 2003/0055470, 2002/0183735, 2001/0014805; and WO2005/067791. US2004/0186468 describes a method and system for treating disorders in parts of the body. Figure 1 shows a system in which barbed ends of electrodes are designed to grab the tissue of a bladder neck and upper urethra in a claw-like manner and bunch it together so that when energy is delivered through the electrodes to the bunched tissue, shrinkage occurs in the areas surrounding the treatment element. Figure 3 shows a system in which a treatment balloon includes a flexible basket structure and a set of surface electrodes evenly distributed on the basket like structure. In use of this system, the treatment balloon is inflated within the bladder and electrodes are selected using an electrode selection and control switch. The release of energy by the electrodes creates a pattern of lesions in the new mucosal or sub-mucosal tissues of the bladder or portions thereof and the affected area shrinks and is relatively strengthened so as better to retain urine.

US2011/0301662 describes an apparatus including a bladder stimulator based on a modified Folev catheter. The stimulator comnrises an inflating balloon section. Ontionallv the stimulator has at least one electrode attached in proximity to the inflating balloon. Further prior art is disclosed in WO 2005/067791 A1. Additional state of the art in the meaning of Art. 54(3) EPC is disclosed in WO 2013/016590 A1. Background art is disclosed in WO 2013/052852 A1 and WO 2013/052848 A1.

### SUMMARY OF THE INVENTION

The present invention is set out in the appended claims. It is the inventors' belief that by applying controlled ablation in the urinary bladder, conductivity within the organ can be modified so as to treat urinary disorders. Accordingly, systems and devices for treating a hollow bodily organ, particularly a urinary bladder for overactive bladder, are disclosed. In many embodiments, a predetermined pattern of tissue regions having reduced electrical propagation is created. These regions of reduced electrical propagation will typically affect the electrical and/or mechanical properties of the bladder to treat overactive bladder, for example, by reducing the occurrence of the undesirable muscle spasms that cause the disorder.

Described herein is also a method , the method not forming part of the claimed invention, of treating a urinary disorder in a bladder. A predetermined pattern of tissue regions having reduced electrical propagation is created in an inner wall of the bladder. Creating these tissue regions with reduced electrical propagation modifies at least one of a mechanical or an electrical property of the bladder.

The predetermined pattern of tissue regions having reduced electrical propagation can have a variety of therapeutic functions. The tissue regions having reduced electrical propagation can achieve at least one of preventing, attenuating, or slowing dissemination of electrical activity within bladder tissue. Additionally or alternatively, the tissue regions having reduced electrical propagation can achieve at least one of preventing, attenuating, or modifying the transfer of mechanical forces through the bladder. The tissue regions having reduced electrical propagation can also decrease bladder smooth muscle contraction caused by aberrant electrical activity. Any one or more of these therapeutic functions can reduce or prevent symptoms of overactive bladder or other bladder conditions.

The predetermined patterns of tissue regions having reduced electrical propagation can have a variety of configurations. The tissue regions may be arranged to have a long axis parallel to a long axis of the bladder when full. The tissue regions may be arranged to have a long axis transverse to a long axis of the bladder when full.

The tissue regions may be selected to electrically isolate one or more anatomical regions in the bladder such as the ureteral orifice, the uretero vesical junction, the trigone area, the bladder dome, or the bladder outlet. The tissue regions having reduced electrical propagation will typically have a depth sufficient to attenuate, slow, or even block electrical propagation through the tissue. For example, the tissue regions having reduced electrical propagation can extend through the urothelium, through the urothelium and the suburothelium, through the urothelium and suburothelium and at least a part of the detrusor, or through an entire wall of the bladder, i.e., be transmural.

In many cases, the predetermined pattern of tissue regions having reduced electrical propagation may comprise a plurality of tissue lines having reduced electrical propagation. These tissue lines may provide partial or complete barriers to electrical propagation from the ablated tissue regions to adjacent tissue regions. These tissue lines may come in a variety of configurations, including circumferential lines, longitudinal lines, parallel lines, crossing lines, straight lines, serpentine lines, continuous lines, zig-zag lines, and broken lines. The tissue lines may have a width in a range from 1 mm to 10 mm and be separated from one another by a distance in a range from 10 mm to 150 mm. The tissue lines may be arranged such that contact between the issue lines is minimized when the bladder is collapsed. The tissue lines may be arranged to avoid one or more anatomical regions selected from a group comprising the ureteral orifice, the uretero vesical junction, the trigone area, the bladder dome, or the bladder outlet.

In many cases, foci of aberrant electrical activity in the bladder are first located before the predetermined pattern of tissue regions having reduced electrical propagation is created. The predetermined pattern of regions having reduced electrical propagation will then correspond to the located foci of aberrant electrical activity. The foci of aberrant electrical activity in the bladder may be located in many ways, for example, by imaging or visualizing induced contraction of the bladder or by mapping electrical activity in the bladder with a plurality of electrodes of a device introduced into the bladder cavity. The same device may in some cases be used both for mapping the electrical propagation patterns prior to (and in some cases after) treatment and to create the predetermined pattern of tissue regions having reduced electrical propagation. At least a portion of the mapping (and optionally treatment) device introduced into the bladder cavity may be configured to conform to the shape of the inner wall of the bladder.

The predetermined pattern of tissue regions having reduced electrical propagation may be created in many different ways. The tissue regions may be created by at least one of RF ablation, cryoablation, photoablation, microwave energy, use of a chemical agent, ultrasound energy, and mechanical scoring. The predetermined pattern of tissue regions having reduced electrical propagation can be created by placing a tissue modification structure of a device introduced into the bladder cavity at or near a target site in the inner wall of the bladder and moving one or more active elements of the tissue modification structure in a predetermined manner. For example, at least a portion of the active element may be rotated within the bladder to create a continuous tissue region having reduced electrical propagation. The active element may also be moved in other ways such as by translation along the longitudinal axis of the bladder or transverse to the axis. At least a portion of the tissue modification structure near the active element may be configured to conform to the shape of the inner bladder wall. The predetermined pattern of tissue regions having reduced electrical propagation can also be created by placing a plurality of tissue modification structures of a device introduced into the bladder cavity at or near a plurality of target sites in the inner wall of the bladder and applying energy through a plurality of active elements of the tissue modification structures. Energy may be applied by the plurality of active elements simultaneously, selectively, or sequentially. Energy may be applied by the active elements without having to reposition the plurality of tissue modification structures after they have been placed at or near the plurality of tissue sites. Often, the plurality of tissue modification elements is configured to conform to the shape of the bladder cavity. The predetermined pattern of tissue regions having reduced electrical propogation may be created under visualization, including direct visualization of the interior of the bladder through a cystoscope or an ureteroscope. Alternatively visualization can be performed using a video camera, optic fiber, or other means which may be part of the device or be inserted through the at least a portion of the device.

Described herein is a device for treating a urinary disorder in a bladder. The device comprises a shaft, a tissue modification structure, and means for creating a predetermined pattern of tissue regions having reduced electrical propagation in the inner wall of the bladder to modify at least one of a mechanical or an electrical property of the bladder. The shaft is advancable through a urethra of a patient to reach the bladder. In examples not according to the claimed invention, the shaft could be configured to be advanced laparoscopically or by other minimally invasive procedures through a patient's abdominal wall. The tissue modification structure is coupled to a distal end of the shaft. At least a portion of the tissue modification structure is adapted to contact and conform to at least a portion of an inner wall of the bladder. The means for creating the predetermined pattern of tissue regions having reduced electrical propagation can be configured so that the modified tissue regions created will typically have a depth sufficient to attenuate, slow, or even block electrical propagation through the tissue. For example, the modified tissue regions may extend through the urothelium, through the urothelium and the suburothelium, the urothelium and suburothelium and at least a part of the detrusor, or through an entire thickness of the wall of the bladder. Alternatively, the tissue region treated includes the suburothelium and some of the detrusor muscle, sparing the urothelium (also known as a "skip lesion").

The means for creating the predetermined pattern of tissue regions having reduced electrical propagation can be configured so that the predetermined pattern of tissue regions have a variety of therapeutic functions and configurations. Creating these tissue regions can achieve at least one of preventing, attenuating, or slowing dissemination of electrical activity within bladder tissue. Alternatively or in combination, creating these tissue regions can achieve at least one of preventing, attenuating, or modifying the transfer of mechanical forces through the bladder. The tissue regions can decrease bladder smooth muscle contraction caused by aberrant electrical activity.

It may be understood that the treated areas can have other altered biological activities, including altered paracrine activity, altered membrane potential, altered excitability, altered response to neural or chemical activation, altered stretch responses, and/or altered electrical conductivity.

The means for creating the predetermined pattern of tissue regions having reduced electrical propagation are configured to create a plurality of tissue lines having reduced electrical propagation. The plurality of tissue lines having reduced electrical propagation can comprise at least one of circumferential lines, longitudinal lines, parallel lines, crossing lines, straight lines, serpentine lines, continuous lines, zig-zag lines, and broken lines. The tissue lines may have a width in a range from 1 mm to 10 mm and be separated from one another by a distance in a range from 10 mm to 150 mm. The tissue lines may be arranged such that contact between the tissue lines is minimized when the bladder is collapsed. The tissue lines can be arranged to avoid one or more anatomical regions selected from a group comprising the ureteral orifice, the uretero vesical junction, the trigone area, the bladder dome, or the bladder outlet.

The bladder wall contact element will typically comprise a wire cage shaped to conform to the inner wall of the bladder. The wire cage may comprise one or more mapping electrodes for locating foci of aberrant electrical activity in the bladder. The located foci of aberrant electrical activity in the bladder may correspond to the predetermined pattern of tissue regions having reduced electrical propagation. The wire cage may also carry one or more active elements for reducing electrical propagation in tissue. The one or more active elements may be adapted to create the pattern of electrically isolated areas by at least one of RF ablation, cryoablation, photoablation, microwave energy, use of a chemical agent, ultrasound energy, and mechanical scoring. The wire cage may be moveable within the bladder to move the one or more active elements to create the predetermined pattern. The one or more active elements may be adapted to deliver energy to the inner wall of the bladder simultaneously, sequentially, or selectively.

The bladder wall contact element may comprise an elongate curved element shaped to conform to at least a portion of the inner wall of the bladder. The curved element may be electrically coupled to a power generator at a plurality of points along the elongate curved element. The means for creating the predetermined pattern of electrically isolated areas may comprise one or more active disposed on the elongate curved element. The one or more active elements may be adapted to create the pattern of electrically isolated areas by at least one of RF ablation, cryoablation, photoablation, microwave energy, use of a chemical agent, ultrasound energy, and mechanical scoring. The elongate curved element may be movable to move the one or more active elements to create the predetermined pattern.

The device may further comprise an expandable element coupled with the bladder wall contact element. The expandable element is expandable to press the tissue modification structure against the inner wall of the bladder.

Described herein is a system for treating a urinary disorder in a bladder. The system comprises the aforementioned device and a means for visualizing the bladder as the predetermined pattern of electrically isolated areas is created in the bladder. The means for visualizing the bladder may comprise a cystoscope or an ureteroscope, or other visualization means which may be part of the device or is inserted through the at least a portion of the device.

While many embodiments disclosed herein are described as related to or configured for the treatment of a bladder for overactive bladder, the devices described herein may also find use for other hollow bodily organs. Accordingly, described herein is a method, the method not forming part of the claimed invention, of treating a disorder in a hollow bodily organ. A predetermined pattern of tissue regions having reduced electrical propagation is created in an inner wall of the hollow bodily organ to modify at least one of a mechanical or an electrical property of the organ. The hollow bodily organ may comprise a bladder, a bronchus, a bronichiole of the lung, a stomach, a colon, a large intestine, a small intestine, a kidney, a vagina, a uterus, a fallopian tube, an esophagus, a gall bladder, and the like. The reduced electrical propagation regions can be applied to reduce bladder over-activity when applied to bladder, bronchial hyper-reactivity (asthma) when applied to bronchus or bronchiole, gastric peristaltic motions when applied to stomach, irritable bowls (as in irritable bowel syndrome) when applied to colon or intestines, reflex fluid retention when applied to kidney, vaginismus when applied to vagina, premature contractions when applied to uterus or fallopian tube, and esophageal spasm and/or gastroesophageal reflux disease when applied to the esophagus.

Described herein is a device for treating a disorder in a hollow bodily organ. The device comprises a shaft advancable through a bodily passage of a patient to reach the hollow bodily organ, a tissue modification structure coupled to a distal end of the shaft, and means for creating a predetermined pattern of tissue regions having reduced electrical propagation in the inner wall of the hollow bodily organ to modify at least one of a mechanical or an electrical property of the hollow bodily organ. At least a portion of the tissue modification structure is adapted to contact and conform to at least a portion of an inner wall of the hollow bodily organ. The hollow bodily organ to be treated is selected from the group comprising a bladder, a bronchus, a bronichiole of the lung, a stomach, a colon, a large intestine, a small intestine, a kidney, a vagina, a uterus, a fallopian tube, an esophagus, a gall bladder, and the like.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 is a cut-away view of an ablation device adapted for insertion through the human urethra, according to many embodiments;
Figure 2 is a perspective view of a longitudinal electrode arrangement for the device of Figure 1, according to many embodiments;
Figure 3 is a perspective view of a circumferential electrode arrangement for the device of Figure 1, according to many embodiments;
Figure 4 is a perspective view of an electrode arrangement, not falling within the scope of the invention claimed, to produce a pattern of distributed spots for the device of Figure 1, according to many embodiments;
Figure 5 is a perspective view of the inner wall of a hollow bodily organ having an ablation pattern ablated thereon by the device of Figure 4;
Figure 6 is a perspective view of a non-continuous circumferential electrode arrangement for the device of Figure 1, according to many embodiments;
Figure 7 is a perspective view of a serpentine electrode arrangement for the device of Figure 1, according to many embodiments;
Figure 8 is a top view of ablation lines, according to many embodiment;
Figure 9a is a cut-away view of an ablation device having a cooling mechanism and adapted for insertion through the human urethra, according to many embodiments;
Figure 9b is a cut-away view of another ablation device having a cooling mechanism and adapted for insertion through the human urethra, according to many embodiments;
Figure 10 is a perspective view of an ablation device comprising a plurality of exterior fluid channels and adapted for insertion through the human urethra, according to many embodiments;
Figure 11 is a perspective view of a parallel electrode arrangement for the device of Figure 1, according to many embodiments;
Figure 12 shows a flow chart of a method to treat a hollow organ, not according to the claimed invention;
Figure 13 is a cut-away view of a bladder having parallel ablation lines created thereon, according to many embodiments;
Figure 14 is a cut-away view of a bladder showing safe zones to avoid ablation, according to many embodiments;
Figure 15 is a perspective view of a rotatable ablation device , not according to the claimed invention;
Figure 16 is a cut-away view of a bladder having a catheter with a light source placed therein, according to many embodiments;
Figure 17 is a perspective view of a tool for drawing ablated or otherwise modified tissue lines within a hollow organ, not according to the claimed invention;
Figure 18 is a cut-away view a bladder having an ablation device with a cage-like structure advanced therein, according to many embodiments;
Figure 19 is a cut-away view of a bladder having another ablation device with a cage-like structure advanced therein, according to many embodiments;
Figure 20 is a cut-away view of a bladder having yet another ablation device with a cage-like structure advanced therein, according to many embodiments;
Figure 21 is a cut-away view a bladder having an ablation system advanced therein, not according to the claimed invention;
Figure 22 is a cut-away view of the bladder showing a pattern of ablation lines created therein, according to many embodiments;
Figure 23 is a cross-sectional view of an ablation tool having a pre-curved distal portion, not according to the claimed invention;
Figures 24A to 24D are cross-sectional views of various distal tips of the ablation tool of Figure 23, not according to the claimed invention ;
Figure 25A is a cut-away view of a tip of an ablation tool, not according to the claimed invention;
Figure 25B to 25F are schematics of a method of using the ablation tool of Figure 25A to create an ablation pattern, not according to the claimed invention;
Figure 25G is a top view of an ablation pattern that uses short curved lines to create a continuous isolation front that can be created using the ablation tool of Figure 25A and other ablation tools including those of Figures 17, 21, and 23, not according to the claimed invention;
Figures 26A to 26C show a view of tip of an ablation tool through a scope, not according to the claimed invention;
Figure 27A is a cross-sectional view of an ablation tool having a guide element and placed in the bladder, not according to the claimed invention;
Figure 27B is a cut-away view of the ablation tool of Figure 27A positioned near the bladder wall, not according to the claimed invention;
Figure 28A is a cut-away view of an ablation tool having a distal balloon placed within the bladder, according to many embodiments;
Figure 28B is a cut-away view of the ablation tool of Figure 28A expanded against the inner wall of the bladder, according to many embodiments;
Figure 29A is a schematic drawing of an electrical system of an ablation tool, according to many embodiments;
Figure 29B is a schematic drawing of a set of electrodes positioned on a non-conductive struts, according to many embodiments; and
Figure 29C is a schematic drawing of a strut having a set of alternately electrically conductive and non-conductive segments, according to many embodiments.

### DETAILED DESCRIPTION OF THE INVENTION

Sytems and devices for treating a hollow bodily organ, particularly a urinary bladder for overactive bladder, are disclosed.

Devices for tissue ablation in hollow organs are described. In many embodiments, tissue ablation regions are created within the treated organ, thus preventing, attenuating, or slowing dissemination of the electrical activity within the tissue. These regions will typically be in the form of lines. Such lines or regions of reduced electrical conductivity or propagation may be described as electrical isolation lines or regions, although complete electrical isolation may not always be achieved or desired. In many embodiments, creating tissue ablation lines or regions within the treated organ can prevent, attenuate, or modify the transfer of mechanical forces through the organ. In some embodiments, ablation lines or regions disperse mechanical forces more evenly, effectively preventing certain cells or tissue regions from being extremely stretched. In some embodiments, the lines and the resulting scarring are induced in a hollow organ, such as a bronchiole or a urethra, to prevent pathological contraction of the organ.

In embodiments according to the invention, the electrical isolation regions or regions of reduced electrical propagation are created in a human urinary bladder. The creation of such lines or regions can decrease bladder smooth muscle contraction caused by aberrant electrical activity in the bladder wall, alleviating the symptoms associated with overactive bladder and/or prostatism.

examples not according to the invention, electrical isolation regions or regions of reduced electrical propagation can also be created in other hollow bodily organs.

The electrical isolation regions or regions of reduced electrical propagation can be created within a human bronchial tree, to prevent synchronized and generalized bronchial constriction, such as those occurring in an asthmatic attack. In some examples , the ablation lines are created in a bronchiole to isolate some alveolar spaces from the rest of the bronchial tree.

The electrical isolation regions or regions of reduced electrical propagation can be created in a human uterus to prevent unwanted uterus contractions that might disturb the normal course of a pregnancy.

The electrical isolation regions or regions of reduced electrical propagation can be created within a human stomach to slow gastric emptying and reduce the weight of the subject.

The electrical isolation regions or regions of reduced electrical propagation can be created within a human colon to alleviate symptoms of erratic GI activity such as irritable bowel syndrome.

Figure 1 is a schematic of a device 100 adapted for insertion through the human urethra according to embodiments of the invention. The device 100 comprises an inflatable balloon 110 and a flexible shaft 120 coupled to the balloon 110. The balloon 110 may be collapsed to reduce the profile of the device 100. The flexible shaft 120 can have sufficient stiffness so that the device 100 can be advanced through the human urethra when in reduced profile. The flexible shaft 120 comprises a urine lumen 122 and an inflation lumen 126. The urine lumen 122 is open at a distal port 124 near the proximal end of the balloon 110 to collect urine within the bladder and divert it out. The inflation lumen 126 is open at a port 128 within the balloon 110 to deflate or inflate the balloon 110. The device 100 further comprises an electrical lead 130 leading to and powering one or more electrodes 132 disposed on the surface of the balloon 110. The balloon 110 may be configured to conform to the shape of the bladder when the balloon 110 is expanded. The balloon 110 may be pre-shaped to be oval and somewhat curved anteriorly. When the balloon 110 is expanded, the one or more electrodes 132 will typically contact the inner wall of the bladder. The one or more electrodes 132 can be used to ablate the inner wall of the bladder and in some cases to also electrically map the bladder. The one or more electrodes 132 may be arranged on the outer wall of the balloon 110 in a predetermined ablation pattern. The ablated tissue regions in the inner wall of the hollow organ may have reduced electrical propagation.

In many embodiments, the ablation device described herein, including the device 100, may be used to ablate localized areas of tissue that express aberrant electrical activity or have other undesirable characteristics. For example, foci of aberrant electrical activity in the urinary bladder may be identified by recording or mapping from electrodes 132 located in the outer wall of the balloon 110. This may be followed by ablation of the identified foci by the same electrodes 132 or via other means. The electrodes 132 may be energized selectively to create a desired ablation pattern based on the identified foci of aberrant electrical activity. For example, embodiments may provide a system for ablating a hollow bodily organ such as the bladder, the system comprising the device 100 and a processor coupled to the device 100. The processor may be configured to run code to cause the device 100 to electrically map the bladder when placed therein, to further determine a predetermined ablation pattern based on the mapped electrical activity, and to further operate the device 100 to selectively energize the electrodes 132 to create the ablation pattern on the inner wall of the bladder. Other means may be provided to create the desired ablation pattern determined by the mapping electrodes 132.

The foci of aberrant electrical activity may also be identified by other means. In some embodiments, the foci are identified by an urodynamic study involving bladder contraction that is coupled to an imaging study (ultrasound, dynamic CT, etc.) visualizing the contraction of the bladder. In these embodiments, the zones that lead the contraction (i.e., tissue regions that contract before the rest of the bladder or more than the rest of the bladder) will be the preferred site for ablation. In other embodiments, an ultrasound study of the bladder is conducted to identify anatomical characteristics that may be preferred ablation sites. In some embodiments, wall thickening areas are targeted.

In some embodiments, residual volume is maintained in the bladder after the ablation for a period of at least 3 hours, to avoid adhesions.

Referring back to Figure 1, the electrodes 132 disposed over the outer wall of the balloon 110 may be configured to ablate the inner wall of a hollow bodily organ such as the bladder to create a variety of patterns. The lines of ablation may be circumferential or longitudinal, parallel or crossing, straight or serpentine, continuous or broken.

Figure 2 shows an electrode arrangement wherein the electrodes 132 are arranged longitudinally on the balloon 110 to contact the inner wall of the hollow target organ so as to be able to create longitudinal ablation lines within a hollow target organ such as the bladder. Longitudinal ablation lines can allow for uninterrupted conduction from the bladder dome downwards while interrupting conduction from one bladder meridian to others. As shown in Figure 2, the longitudinally arranged electrodes 132 do not intersect with one another but may intersect with one another in other embodiments. Such intersecting ablation lines may serve to electrically isolate selected zones of tissue, for example an anatomic structure such as the ureteral orifice, uretero-vescical junction, the trigone area, the bladder dome, or the bladder outlet.

Figure 3 shows an electrode arrangement wherein the electrodes 132 are arranged circumferentially on the balloon 110 so as to contact the inner wall of the hollow target organ to create continuous circumferential ablation lines within a hollow target organ such as the bladder. As shown in Figure 3, the circumferentially arranged electrodes 132 do not intersect with one another but may intersect with one another in some other embodiments. Such intersecting ablation lines may serve to isolate selected zones of tissue as described above.

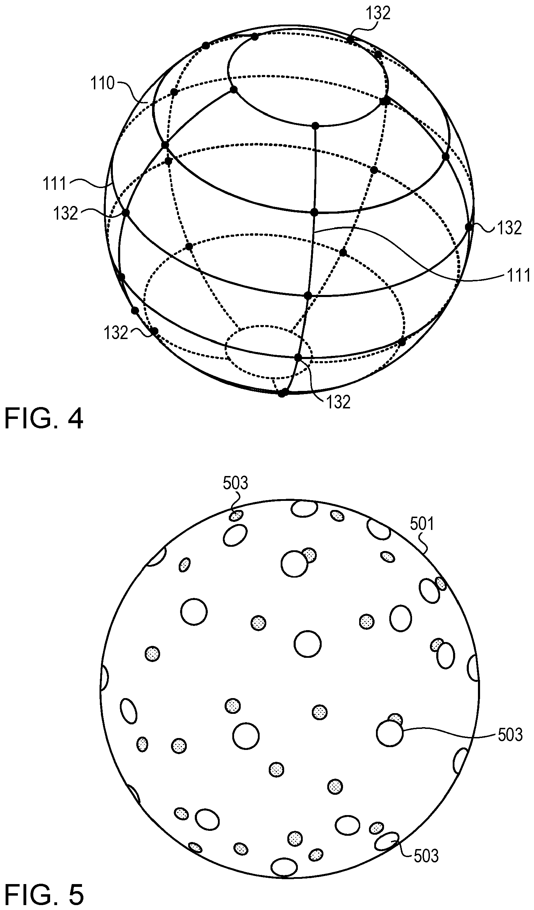

Figure 4 shows an electrode arrangement, not falling within the scope of the invention claimed, wherein the electrodes 132 are supported by a wire cage 111 disposed over the balloon 110 so that their ablating ends are distributed over the area of the wire cage 111. The electrodes 132 arranged in such a manner can create a pattern of ablated tissue spots 503 on the inner wall 501 of a hollow bodily organ such as the bladder as shown in Figure 5. A structure similar to the wire cage 111 shown in Figure 4 where all the wires of the cage are ablating electrodes may be used to create a criss-cross pattern over the inner surface of the treated organ. In many embodiments, the electrodes 132 may be adhered onto the surface of the balloon 110. In other embodiments, the device 100 may further comprise a wire cage to support the electrodes 132.

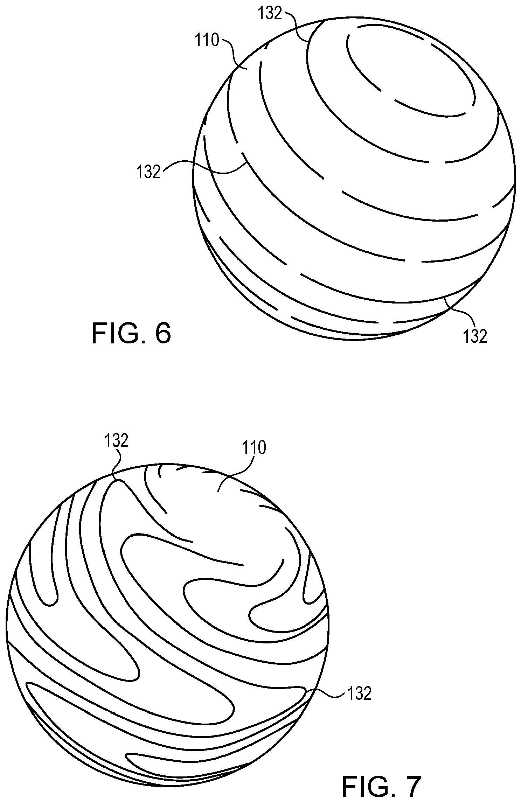

Figure 6 shows an electrode arrangement wherein the electrodes 132 are arranged circumferentially on the balloon 110 so as to contact the inner wall of the hollow target organ to create discontinuous circumferential ablation lines within a hollow target organ such as the bladder.

Figure 7 shows an electrode arrangement wherein the electrodes 132 are arranged on the balloon 110 in a serpentine manner to contact the inner wall of the hollow target organ to create serpentine ablation lines within the hollow target organ such as the bladder.

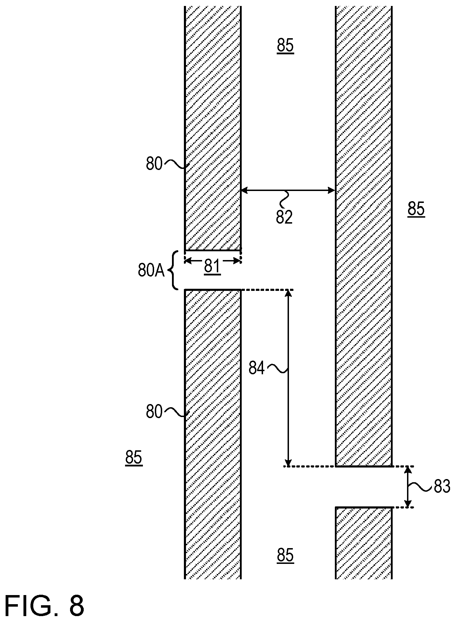

Figure 8 is a top view of ablation lines 80 that can be created by the device 100 in the inner wall 501 of a hollow bodily organ such as the bladder. The ablation lines 80 separate untreated tissue areas 85.

The ablation lines 80 and their arrangement can have a variety of attributes. The thickness 81 of the ablation lines 80 may vary from a minimum of 1 mm to a maximum of 10 mm. Very thin lines may produce incomplete electrical isolation and thus may serve to slow conduction of electrical activity across them without totally preventing its passage. This can prevent aberrant sources from causing synchronized activation of the whole organ, while allowing physiologic activity to spread through the whole organ. Thicker lines can produce complete isolation which may allow the direction of electrical activity in specific pathways, and control over the way electrical activity propagates throughout the organ. The ablation lines 80 can be created to have a depth sufficient to attenuate, slow, or even block electrical propagation through the tissue. For example, the ablation lines 80 may extend through the urothelium, through the urothelium and the suburothelium, the urothelium and suburothelium and at least a part of the detrusor, or through an entire wall of the bladder.

In many embodiments, the width 81 of the ablation line is set to allow regeneration of the transitional epithelium before significant fibrosis occurs. For example, ablation lines 80 can be approximately 3 mm wide in an empty bladder and the epithelium can be expected to regenerate within 10 days before significant fibrosis will occur.

In some embodiments, the ablation lines 80 are of varying thickness, being thicker towards the dome (cranial pole) of the bladder and thinner when nearing the bladder outlet, especially if ablation is applied when the bladder is expanded.

As shown in Figure 8, generally parallel ablation lines 80 may be separated from each other by a distance 82 which may vary between 10 mm to 150 mm. This distance 82 will typically also be the width of the electrical conduction or propagation pathway. Axially adjacent ablation lines 80 may be separated from each other by a distance 83 which may vary between 1 mm and 20 mm. This distance 83 may serve as a bridge between parallel conduction pathways. Such bridges between parallel groups of ablation lines may be axially separated from each other by a distance 84. Like the thickness 81 of the ablation lines 80, the separation distances 82, 83 between the ablation lines 80 and the separation distance 84 between bridges may also affect the ability of electrical activity to propagate between isolated areas or strips of the organ's wall.

In some embodiments, the ablation lines 80 are configured so that contact between ablation zones is minimized, even when the organ is collapsed.

In some embodiments, the dome of the urinary bladder is spared from ablation. In some embodiments, an approximately circular area of the dome is spared, having a diameter of approximately 25 mm in the expanded bladder.

According to the invention , short ablation lines 80 are separated from each other in an expanded bladder, and approximated to become effectively continuous only when the bladder volume is low. In these embodiments, effective bladder contractions can normally occur in the distended bladder, but not in an empty bladder.

Lines of ablated tissue 80 or tissue lines having reduced electrical propagation can be created in many ways, including the use of heat, cold, laser light, microwaves, chemicals, drugs, and more. In some embodiments, the energy applied is electromagnetic energy at radio frequency (RF) through conductive surfaces such as the electrodes 132 of device 100. In some embodiments, the energy applied is monopolar or bipolar and delivered through the conductive surfaces such as the electrodes 132 of device 100. Energy can be produced by an external device and conveyed to the tissue by the catheter device 100, for example through lead 130 as shown in Figure 1. Typically, energy is applied only once or only once every 30 seconds or more to allow cooling of the device 100. The energy applied may be sufficient to cause a transmural lesion.

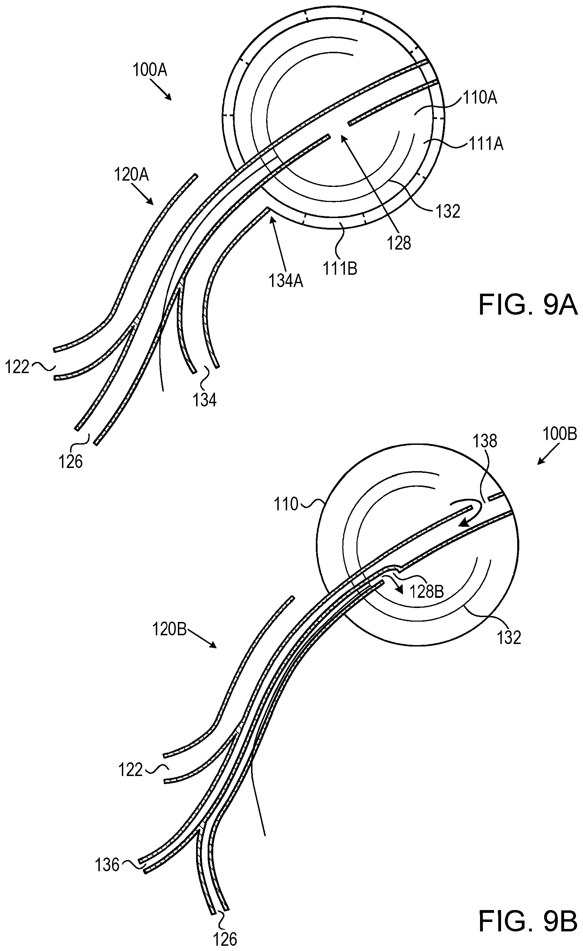

The delivery of energy, particularly electromagnetic and RF energy, typically produces heat. Accordingly, the energy delivery device may need to be cooled to avoid inadvertent damage to the tissue of the organ wall. Also, cooling may protect the device from damage or malfunction. Figure 9a shows an embodiment of an ablation device 100a which can be cooled. The ablation device 100A is generally similar to the device 100 described above. The flexible shaft 120A of the ablation device 100A further comprises a cooling fluid lumen 134 for the introduction and removal of a cooling fluid such as cooled saline. The balloon 110A comprises an inner compartment 111A for inflation and an outer compartment 111B which can be filled with the cooling fluid to cool the balloon 110A when the electrodes 132 have heated. The inflation lumen 126 is open to the inner compartment 111A at the port 128 and the cooling fluid lumen 134 is open to the outer compartment 111B at the distal end 134A of the cooling fluid lumen. Figure 9B shows an embodiment of an ablation device 100B which can be cooled by circulating the fluid which keeps the balloon 110 inflated. The ablation device 100B is generally similar to the device 100 described above. The flexible shaft 120B of the ablation device 100B comprises an inflation lumen 126 to inflate the balloon 110 and a suction lumen 136 to deflate the balloon. Cooled inflation fluid can be introduced into the balloon 110 through the inflation lumen 126 open at the port 128B within the balloon 110. At the same time, inflation fluid which has been warmed by the use of the electrodes 132 can be removed from the balloon 110 through the suction lumen 126 open at the port 138.

In some embodiments, the energy is transmitted to the ablation device placed within the hollow bodily organ. The device may be a metallic device and the energy may be transmitted through magnetic fields. In other embodiments, the energy is ultrasound that is reflected and focused by air channels in the device.

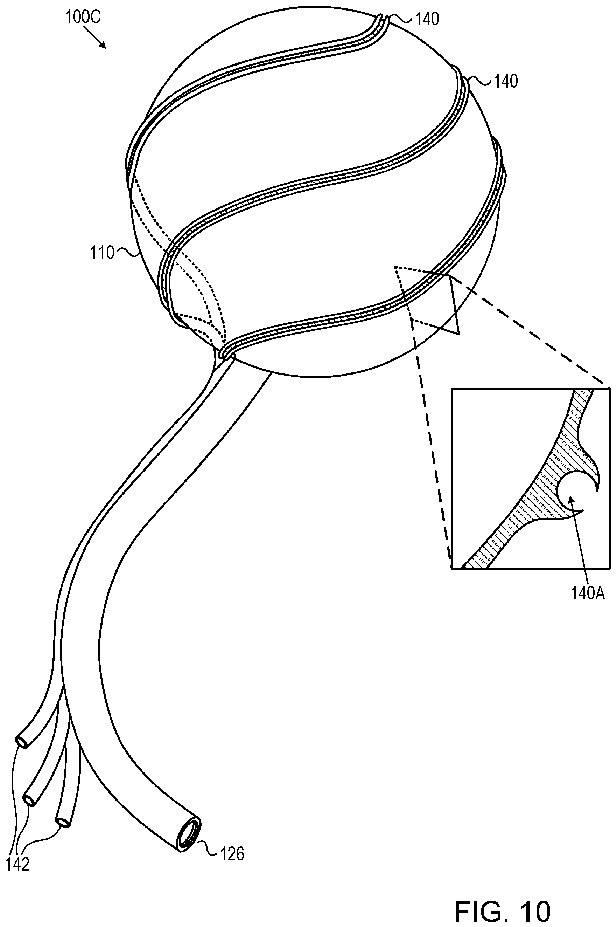

Figure 10 shows an ablation device 100C generally similar to device 100 described above. The ablation device 100C has a plurality of exterior fluid channels 140 placed on the outer surface of its inflatable balloon 100C. Fluid can be introduced into and removed from the exterior fluid channels 140 through tubes 142. When expanded within a hollow bodily organ, exterior fluid channels 140 contact points in the inner wall of the organ. In some embodiments, hot fluid and/or steam flows through the tubes 142 and the exterior fluid channels 140 to heat the contact points to a temperature that is damaging to the tissue. In some embodiments, condensed gas, such as liquid nitrogen, flows through the tubes 142 and the exterior fluid channels 140 to cool the contact points to a temperature that is damaging to the tissue. In some embodiments, the plurality of exterior fluid channels 140 are inflated to define a guidance channel 140a to guide a separate ablation catheter in specific paths on the surface of the balloon 110. The channels 140 may be closed (tube-like) or open (channel-like).

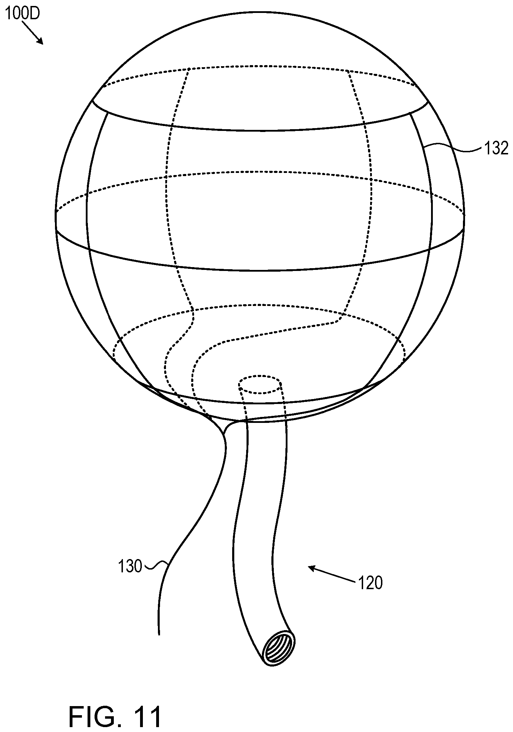

The ablation elements such as electrodes 132 and the fluid channels 140 described above can be configured to contact the inner wall of a hollow target organ in many ways. The conductive surface of the ablation device may be shaped as a blade, with the non-insulated side coming in contact with the tissue being significantly narrower than the side contacting the device. In embodiments not according to the invention, the energy for an isolation or reduced electrical propagation line can be applied only at one or more points, and the lines may be created by rotation and/or movement of the device. The energy for an isolation or reduced electrical propagation line may be applied only at one or more lines, and ablation patterns may be created by rotation and/or movement of parts of the device. For example, as shown by Figure 11, the contact lines may comprise multiple conductive segments or electrodes 132 that are electrically coupled in parallel to avoid temperature gradients along contact lines.

As discussed above, the device 100 can include an inflatable or otherwise expandable member or balloon 110 that is used to approximate the contact points to the tissue in many embodiments. The inflatable or otherwise expandable member 110 may be shaped to conform to the inner wall of the organ when inflated. The balloon 110 may be pre-shaped to the shape of a urinary bladder. The balloon 110 or other expandable member or approximating device may be pre-shaped to best fit the urinary bladder. In some embodiments, the cross section of the upper pole of the balloon is visibly larger than the cross section of the lower pole-the pole closer to the bladder outlet. In other embodiments, the anterior to posterior axis of the balloon 110 is visibly shorter than the up to down axis, and/or the left to right axis. The balloon 10 may have a high compliance and thus low filling pressures and high conformity. In some embodiments, the element to be placed within the hollow organ does not expand but changes shape. For example, the device or parts thereof change their shape from substantially straight or slightly curved to markedly curved.

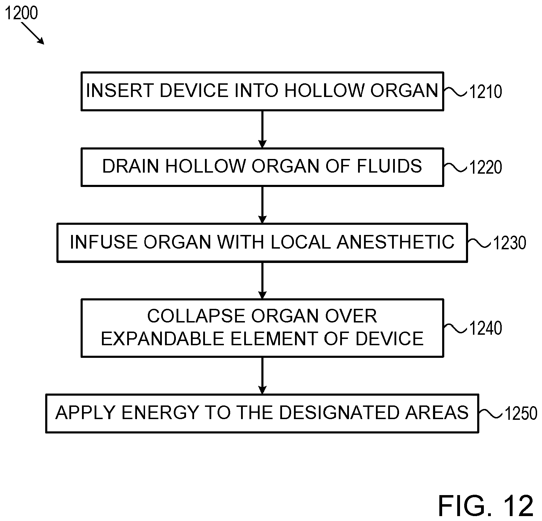

Described herein are methods for creating tissue lines having reduced electrical propagation, said methods not forming part of the claimed invention. Figure 12 shows a flow chart of an exemplary method 1200. In a step 1210, a device such as device 100 or similar is inserted into a hollow organ. In a step 1220, the hollow organ is drained of fluids, e.g., the hollow organ is emptied from liquids or other materials occupying the organ such as urine from a bladder. In a step 1230, the organ is infused with a local anesthetic such as lidocaine. In a step 1240, the organ is collapsed over an expandable element, such as balloon 110, of the device to ensure good contact. The step 1240 may further involve expanding or otherwise changing the shape of the expandable element to conform to the inner wall of the organ. This can be done by draining the fluid in the organ or by applying suction. In a step 1250, energy is applied to the designated areas to create the tissue lines. Prior to the step 1250, the device may also electrically map the bladder to determine an appropriate ablation pattern. A system may be provided for performing the method 1200. The system can comprise a processor configured to run code to operate an ablation device 100 or similar devices as well as other accompanying devices to implement the method 1200 or the line in a hollow target organ of a patient.

In some embodiments, the step of expanding the expandable member is preceded by a step of pharmaceutically expanding the organ. For example, bronchodilators for bronchial application, muscle relaxants for bladder applications, etc. In some embodiments, the step of expanding the member is followed by a step of pharmacologically or otherwise contracting the hollow organ to be treated. For example, urinary bladder contraction can be induced once the device is in place.

In some embodiments, ablation is applied to the urinary bladder wall when the bladder volume is minimized, e.g., by draining, and thus the bladder wall thickness is maximal and the chance for bladder perforation is reduced. In other embodiments, ablation is applied to the urinary bladder wall when the bladder is expanded after so that the bladder wall is thinned and a transmural lesion can be readily achieved.

In some embodiments, the performance of the tissue lines having reduced electrical propagation is tested by stimulation and recording the electrical activity from different sides of the line. The time for signal propagation over the line is measured and success can be defined as a time lag that is at least three times the time lag expected by the distance between the point of stimulation to the reading point divided by the velocity of signal propagation in the specific tissue concerned.

In some embodiments, the creation of the reduced electrical propagation tissue lines is preceded by measuring the intrinsic electrical activity of the organ.

In some embodiments, the reduced electrical propagation tissue lines are zig-zag lines, to increase the length of actual ablated tissue without increase the width of the ablation line.

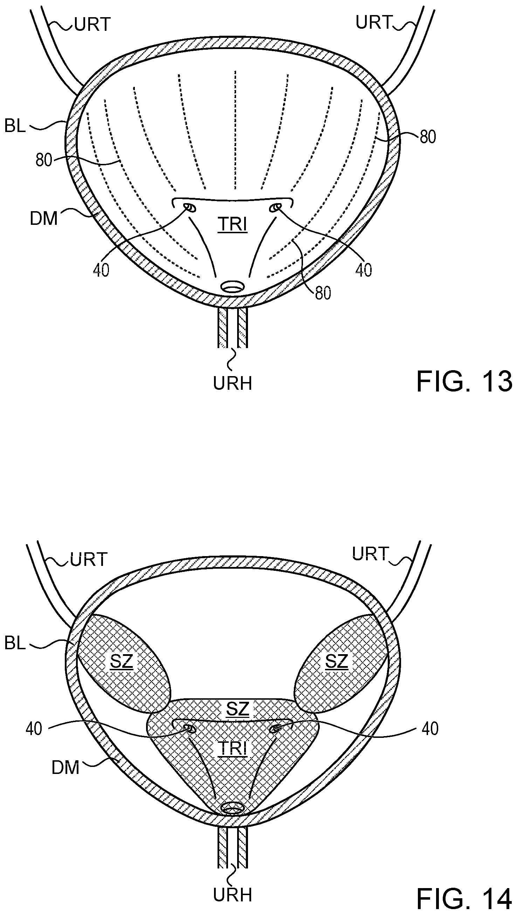

In some embodiments, the ablation lines 80 are parallel lines arranged to avoid one or more anatomical areas such as the trigone area TRI as shown in Figure 13. Figure 13 shows a cut-away view of a bladder BL with such ablation lines 80. Also shown are the detrusor muscle DM, the urethra URH, ureters URT, and ureteral openings UO.

In some embodiments, the device includes a "safe zone" or a "safety zone," where no energy is applied to, in order to protect sensitive areas, such as the ureterovescical orifices in the urinary bladder. This "safe zone" or "safety zone" actively acts as a spacer, planned to displace the energy source from the sensitive areas. Figure 14 shows a cut-away view of a bladder BL showing exemplary safe zones SZ on the inner wall of the bladder BL where no energy is applied.

Many parameters can be measured while the reduced electrical propagation tissue lines are created. The temperature of the device may be monitored. The temperature of the contact points may be monitored. The pressure of the contact points over the tissue may be monitored. The impedance between the device and the tissue may be monitored.

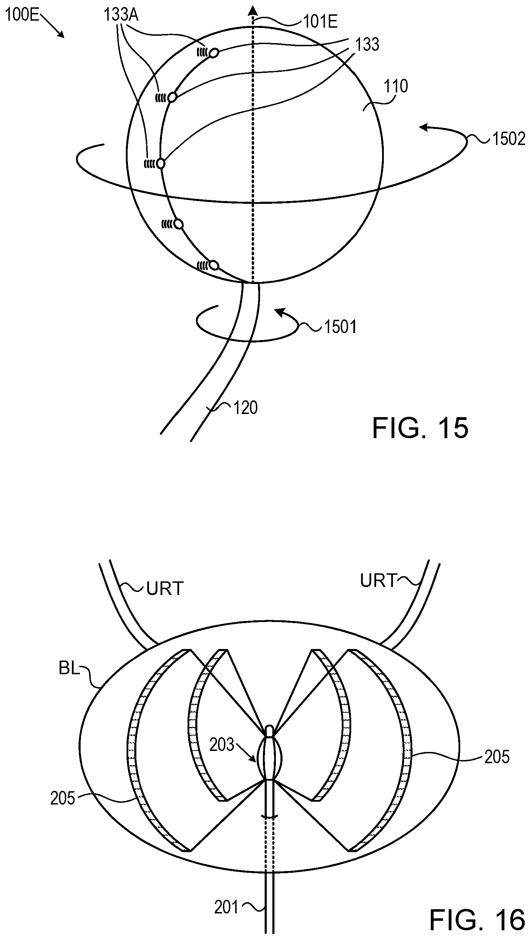

As discussed above, the reduced electrical propagation tissue lines 80 can be created by applying energy through the electrodes 132 disposed on an expanded balloon 110 held stationary within the bladder or, in examples not according to the claimed invention, can be created by rotating the balloon 110 to move tissue modification contacts disposed over the balloon. As shown in Figure 15, an ablation device 100E similar to the ablation device 100 comprises a plurality of electrode contact points 133 disposed over the balloon 110 along a longitudinal line. The balloon 110 can be rotated in a direction 1502 by rotation of the flexible shaft 120 in a direction 1501 to create multiple ablation lines transverse to the longitudinal axis 101e which is aligned with the longitudinal axis of the hollow organ or bladder. The flexible shaft 120 will typically be torquable so that rotation of the shaft 120 can rotate the balloon 110. Figure 15 further shows parts of the paths 133A the electrode contact points 133 travel as the balloon 110 is rotated.

In many embodiments, it is appreciated that direct visualization of the ablation while creating ablation lines is pivotal for procedure safety and flexibility. The hollow organ such as the bladder can be illuminated or otherwise visualized while the lines or regions of reduced electrical propagation are being created. In some embodiments, the lines of modified tissue may be created under visualization using ultrasound, for example, from an ultrasound source advanced into the hollow organ. In some embodiments, light is applied. Figure 16 shows a catheter 201 having a light source 203 disposed on its distal end. The catheter 201 has been advanced through the urethra to position the light source 203 within the bladder BL. The light source 203 illuminates the inner wall of the bladder BL along lines 205 which may correspond to the ablated or otherwise modified tissue lines to be created by the device 100 or others.

Not according to the claimed invention , the light source 203 may even be used to create the lines of modified tissue such as by delivering laser light along the desired lines. The wavelength applied may be between 800 to 1,300 nm, for example 900 nm. Applying such light can be done by optic fibers delivering the energy directly to the bladder wall, or by a prism placed near the light source 203 at the end of the catheter 201 in the center of the bladder BL, which creates the laser light pattern on all the bladder surfaces at once along lines 205.

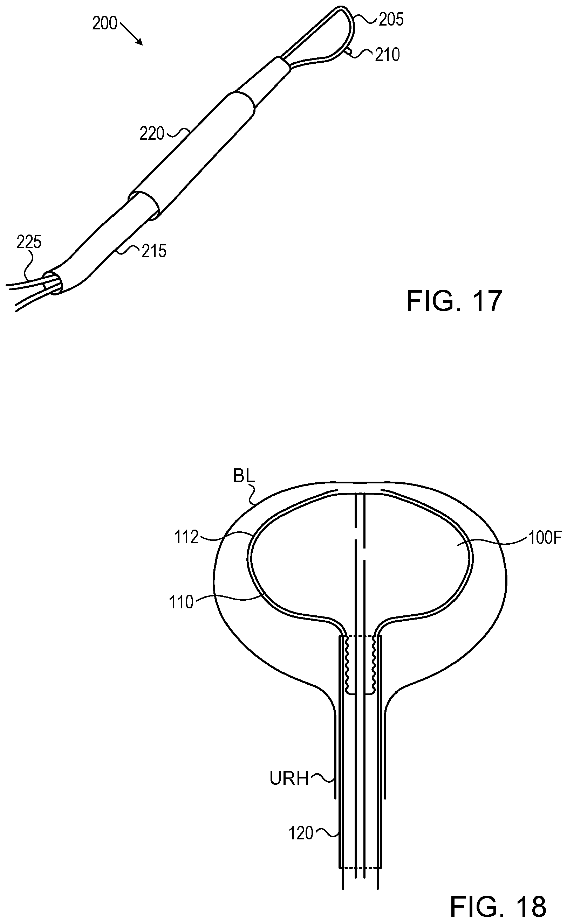

Not according to the claimed invention, the lines of modified tissue may also be drawn on the inner wall of the bladder BL using a tool 200 shown by Figure 17. The tool 200 comprises a curved distal wire portion 205 having an active tissue modification element 210. The curved distal wire portion 205 is curved to protrude in one direction. The tool 200 further comprises an elongate inner shaft 215 and an elongate outer shaft 220 slidably disposed over the elongate inner shaft 215. The elongate outer shaft 220 can be advanced to collapse and cover the curved distal end 205 to reduce the profile of the tool 200 as it is advanced into a hollow organ. The elongate outer shaft 220 can be retracted over the elongate inner shaft 215 to allow the curved distal wire portion 205 to assume its curved shape as shown in Figure 17. If applicable, energy can be transferred to the active element 210 through leads 225 disposed within the elongate inner shaft 215. In some embodiments, the active element 210 comprises an electrode for delivering RF, microwave, heat, or other energy. The lines of ablated or otherwise modified tissue can be created by rotating or translating the tool 200 within the hollow organ. In some embodiments, the inner shaft 215 can be rotated while holding the outer shaft 220 stationary within the urethra to minimize any damage that may be caused to the urethra by such rotation.

In some embodiments, the active element 210 comprises a blade to modify the tissue by cutting or incision. The depth of the incision can be controlled by the choice of the appropriate blade wire out of a selection including several different depths, ranging from 2mm to 8 mm, for example, 4 mm. The depth of the incision is determined by the protrusion of the blade from the surface of the expandable member such as the distal end 205 biased to expand to its curved configuration. Scar tissue formed after the cut can modify the electrical propagation of the surrounding tissue.

In some embodiments, the active element 210 comprises a port for delivering a heated or cooled fluid to ablate tissue.

In some embodiments, the active element 210 comprises a dedicated cup-shaped member that contacts the inner wall of the organ to facilitate the creation of modified tissue lines.

In some embodiments, the elongate tool 200 can be guided by the channels 140 disposed on the expanded balloon 110 of the device 100c shown by Fig. 10, for example, to cut or ablate tissue along the direction of the channels 140. The channel 140 holding and guiding the blade can be covered or roofed along certain areas of the channel 140. The channel 140 may be roofed from the insertion point and up to 3 mm above the bladder outlet. The roofing may be longer on the dorsal aspect of the expandable member than on the ventral side, for example, to prevent dissection in the area of the trigone. The roofing can resume toward the dome of the expandable member, leaving 4 mm or more protected. The curved distal wire portion 205 may be bladed only segmentally. The roofed segments of the guiding channels 140 adapted to face the body of the bladder may be significantly shorter from the exposed segments in these areas, i.e., only one tenth of each channel is roofed in this zone. In other embodiment, the channel supporting the wire 205 is intermittently roofed, to support the wire blade 205 and to create non-continuous lines. In some embodiments, roofed segments along one blade line are in close proximity to the non-roofed segments of a different line so that there is some overlap between lines and the cuts are continuous or near continuous.

The blades can be configured in many other ways. Short blades may be connected, for example by a string, so that several blades can be pulled together as one, while allowing the expandable member to easily deform and adapt to the shape of the bladder. The blades may be positioned to be tangent to the circumference of the device and may be moved to a radial cutting position only when pulled or pushed into the desired position of the line. The necessary blades may be pre-positioned on the device. The blades may be inserted into the device once the device is in position. In some embodiments, the blades protrude from a surface that has a width of at least 3 mm, e.g., 6 mm. This extra width of the surface supporting the blade prevents the blade from "sinking" into the tissue it is cutting. In some embodiments, cutting, scarring, and/or coagulation are facilitated by passing an electrical current through the blades.

In some embodiments, the lines of ablated or reduced electrical propagation tissue are especially crowded in the trigone area which is densely innervated.

In some embodiments, the step of creating the lines is preceded by a step of infusing the bladder with lidocaine or other local anesthetic and/or muscle relaxant and/or anticholinergic agent so that the bladder wall is relaxed and able to stretch.

In some embodiments, in order to facilitate the creation of the lines, the bladder is inflated with an inert gas such as CO₂. Coverage of the ureteral openings for preventing backflow of the pressurized gas is performed by dedicated parts of the device. These can be shaped as plugs entering the orifices or as flat wide covers that are pressed against the bladder wall over the orifices.

In some embodiments, the expandable member such as balloon 110, an introduced gas, and/or an introduced fluid applies pressure on the bladder wall to attenuate the occurrence of edema. In some embodiments, such pressure will be in the range of 10 to 30 cm of water (0.001 bar to 0.03 bar) such as 14 cm of water (0.014 bar).

In some embodiments, special care is taken to minimize the damage to the urothelium when creating the lines. In some embodiments, the member coming in direct contact with the urothelium and adapted to create the lines will be cooled as described above.

While typically reduced compliance of the bladder is associated with overactive bladder symptoms, a method not forming part of the claimed invention treats overactive bladder symptoms by preferentially reducing the compliance of the bladder at certain areas, while allowing other areas to stretch without interruption. In some embodiments, the lines and the resulting scarring and fibrosis are induced in the urinary bladder to change the bladder wall properties so that the compliance of certain areas in the bladder wall is reduced. In some embodiments, lines within the trigone area limit the stretching of the trigone upon bladder filling. In some embodiments, the stretching and contraction of the dome of the bladder is reduced.

In some embodiments, for example as shown in Figure 18, a cage-like structure 112 of a device 100F is used to deliver energy to the bladder wall. An advantage of using such a structure is that it can allow for better and easier visualization of the device and bladder from within the bladder, with a cytoscope, a miniature video camera, or any other optical device. Another advantage is that the cage-like structure can avoid possible damage to the balloon due to warming of the electrodes or tissue. Figure 18 shows the cage-like structure 112 disposed over a deflated balloon 110. The cage-like structure 112 is made of a malleable or superelastic metal and is located at the distal end of the catheter shaft 120.

In use, the catheter shaft 120 is inserted into the bladder and positioned at its center. As the balloon 110 is inflated, it expands the cage-like structure 112 until its struts oppose the bladder wall. Since the balloon 110 and cage 112 are longer in the non-expanded than in the expanded state, part of these structures will be out of the bladder, i.e., could be within the urethra at the time inflation begins. An external sheath, such as the distal part of the shaft 120, prevents expansion of the parts of the balloon 110 and cage 112 that are outside the bladder, until they are gradually pulled into the bladder, as the balloon 110 inflates. After full expansion of the cage 112, the balloon 110 can be deflated.

An advantage of this device 100F is that the device 100F may adapt to the precise anatomical shape of the bladder. Removal of such cage 112 can be done by forcefully pulling it into the rigid shaft 120 at the bladder outlet, such that the cage 112 is compressed and assumes a diameter which allows it to exit.

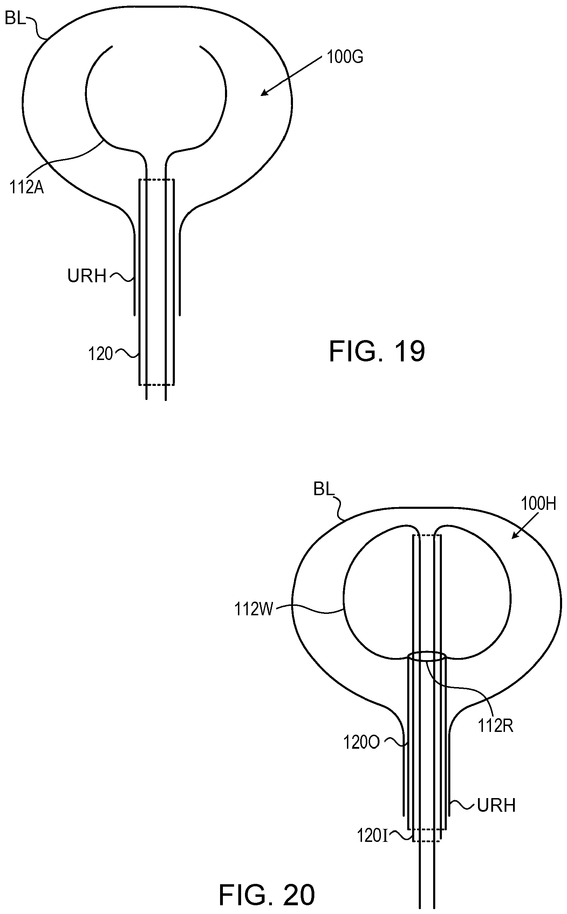

In other embodiments, the ablation or tissue modification device 100G does not utilize a balloon as shown in Figure 19. In these embodiments, the cage-like structure 112A is self-expandable. The cage-like structure 112A can be made of a shape memory metal such as Nitinol and can be pre-shaped to assume the typical anatomy of the bladder. The cage-like structure 112A can be advanced from the lumen of the shaft 112A to expand and retracted into the lumen to collapse it. Alternatively, the cage-like structure 112A could be made of a plastic polymer and electrodes could be attached to it.

Another embodiment of a device 100H similar to the device 100G is shown in Figure 20. The device 100G comprises multiple flexible wires 112W connected to a ring 112R. The wires 112W project forward from the perimeter of the ring 112R, coil back and pass through an inner tube 1201. The ring 112R is connected to the distal tip of an outer catheter tube 1200. The outer catheter tube 1200 can be advanced over the inner catheter tube 1201 which encircles all the wires 112W.

In use, the catheter tube 1201 is placed within the bladder BL with its tip at the bladder outlet. The wires 112W are pushed forward so they arch toward the bladder wall. The inner tube 1201 is pushed toward the dome of the bladder. The inner tube 1201 holds the wires 112W together at the distal side of the formed structure such that its shape can be adapted to that of the bladder BL and the wires 112W come in contact with the bladder wall.

The cage-like structures 112, 112A, 112W described above are preferably continuous with or connected to a cable exiting the bladder through the catheter 120. This connection serves as an anchor for the cage-like device, which also aids in its removal. In addition, the connection allows for transfer of energy such as electromagnetic energy or mechanical energy for example in the form of vibration.

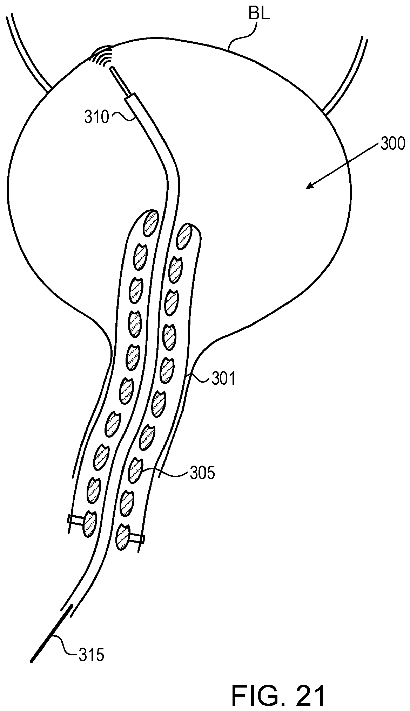

An example not according to the claimed invention is shown in Figure 21 which shows a system 300 for ablating or otherwise modifying tissue in a hollow bodily organ. The system 300 can provide the surgeon with a stable base within the bladder BL from which he can work, while allowing him to control the precise location of the ablation catheter 315. The system 300 comprises a shaft 301 comprised of multiple links 305 that may be pulled together to prevent their relative movement and maintain the shape of the shaft 301. This can be done by a wire going through the links 305 or by an external sheath connected to the last link. A flexible cystoscope 310 of the system 300 can be inserted through the shaft 301. An ablation catheter 315 of the system 300 can be inserted through the cystoscope 310 and directed at any point on the bladder wall.

With the above balloon-less devices, inflation of the bladder may be necessary both for stretching the bladder and for minimizing edema in the wall. As described above, temporary closure of the ureteral orifices may be performed with dedicated parts of the device to prevent backflow during high pressure inflation.

An issue that can be relevant to the embodiments wherein electrodes 132 are embedded in an inflatable balloon 110 is that the length of the electrodes 132 can be greater when the balloon 110 is inflated than when it is deflated. Often, the electrodes 132 accommodate this difference in several ways. The electrodes 132 may be made of an extensible material, for example a thin metal strip of stainless steel, so that when the balloon 110 inflates, they elongate as needed, and remain so. Alternatively, the electrodes 132 could be made of a flexible conductor, such as various graphene based conductors. Still alternatively, the parts or all of the electrodes 132 may be shaped in a delicate zig-zag pattern such that the electrode 132 can straighten out and allow elongation. Lastly, the electrodes 132 may consist of multiple slideable sections that allow elongation while maintaining electrical continuity.

Areas of the cage-like structure 112, 112A and cable or electrodes 132 that do not ablate may be shielded to prevent unintentional ablation or undue transfer of energy to tissues or parts of the device.

In some embodiments, the devices described herein are used to induce bladder auto-augmentation. Thin incisions of the detrusor muscle can allow the creation of bladder diverticuli, where the mucosa and submucosa protrude into the abdominal cavity and the bladder capacity is increased. In some embodiments, the autoaugmentation is achieved using the abdominal approach and in some embodiments, the autoaugmentation is achieved through the urinary bladder. In some of the latter embodiments, the cutting or ablation of tissue is achieved by creating a skip lesion to ablate the underlying detrusor while minimally harming the mucosa. Such skip lesions may be created by various methods known in the art, such as focused ultrasound, cooled RF probes, microwave probes, etc. In other embodiments, the lesions are transmural, and become epithelialized only later, by way of natural epithelial regeneration.

In some embodiments, the lines created are then coagulated to prevent bleeding. In some embodiments, the same element used for ablation is used for coagulation. In some embodiments, when the lines are created surgically, coagulation cautery is applied to the blades to prevent bleeding and facilitate scarring.

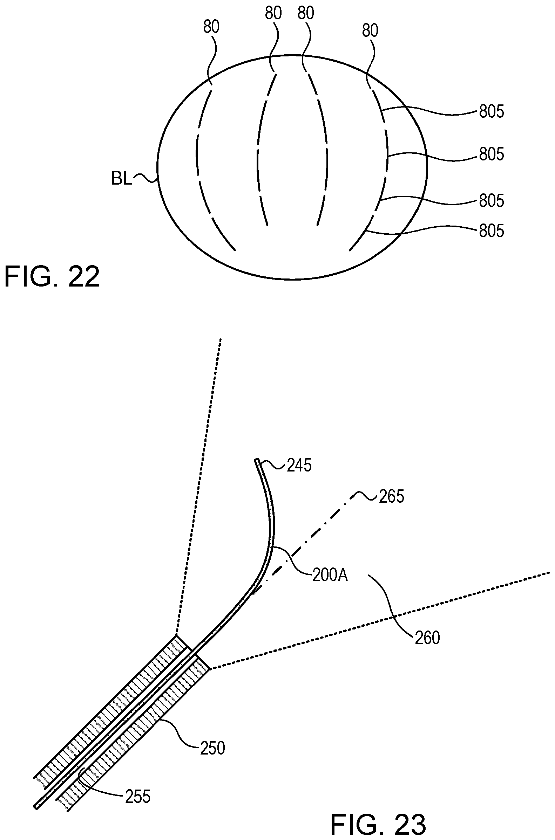

In some embodiments, the ablation or reduced propagation lines 80 are created in short segments 80s at a time in bladder BL as shown in Figure 22. Typically, the length of each section is between 2 cm to 10 cm, preferably 3-4 cm.

Figure 23 shows an ablation tool 200A, not according to the claimed invention, similar to ablation tool 200 described above. In some embodiments, the ablation tool 200A is fitted through the working channel 255 of a standard scope 250 as shown in Figure 23. The ablation tool 200A may have an external diameter of less than 3 mm. The ablation tool 200A can extend beyond the length of the scope 250 for a distance of 2 cm to 10 cm. The ablation tool 200A or part of it is flexible and pre-shaped, so that when extended beyond the working channel of the scope the tool will bend away from the centerline 265 of the scope's field of vision 260. The distal portion 245 of the tool 200A can be pre-shaped as an arc as shown in Figure 23 in some embodiments. In these embodiments, the surface of the tool that comes in direct contact with the bladder, i.e., the electrode, can be pressed against the bladder under direct visualization through the scope 250.

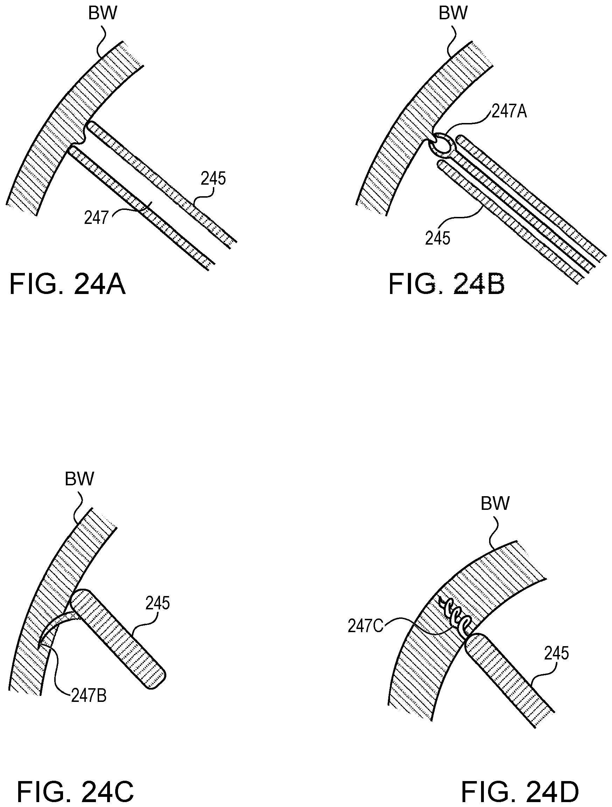

In some examples not according to the claimed invention , part of the tool 200A can be designed to gain traction against the bladder wall BW so that the tool 200a can be maintained at a certain position even if the scope is moved so that continuity of ablation lines can be achieved. Such traction may be created, for example, by none-smooth surfaces in part of the device, applying suction to the bladder wall BW through a channel 247 in the distal portion 245 of the tool 200A as shown in Figure 24A, by "pinching" of the bladder wall BW tissue applied by a miniature pair of tweezers 247A as shown in Figure 24B, by a straight or curved needle 247b extending outward from the distal portion 245 of the tool 200A as shown in Figure 24C, by a spiral needle 247C extending outward from the distal portion 245 of the tool 200A as shown in Figure 24D, or the like.

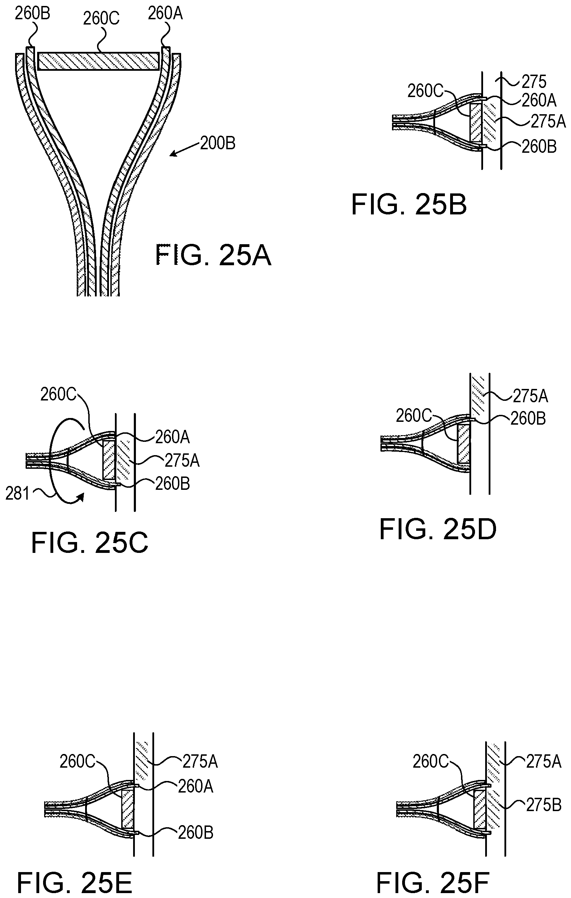

In some examples not according to the claimed invention , line continuity is achieved by rotational movement of the ablation tool, with the so called "anchor" of the electrode being the pivot of rotation. A second "anchor" structure can be positioned on the other side of the electrode segment that creates the segment of ablation. The progression of the tool along the intended ablation line can be achieved by rotational movement of the tool 180 degrees while one "anchor" acts as a pivot, followed at the next step by a 180 degree rotation to the other side, with the other anchor acting as a pivot. This may be achieved by a device 200B as shown in Figure 25A. The device 200B can be collapsed into a low-profile configuration and advanced into the bladder through the scope 250. The device 200B comprises a first extendable and retractable electrode tip 260A and a second extendable and retractable electrode tip 260B, both of which can act as "anchors." The electrode tips 260A and 260B are connected by a wire electrode 260C. The whole device 200B can be rotated about the axis of the first electrode tip 260A or second electrode tip 260B. During ablation, both tips 260A, 260B are extended, serving to anchor the electrodes 260A, 260B and wire 260C in place as shown in Figure 25B. After a first tissue segment 275A is ablated by the electrodes 260A, 260B, the distal tips 260A is retracted and the device 200b is rotated 180 degrees in a direction 281 around the tip 260B which serves as an "anchor" as shown in Figures 25C and 25D. The retracted tip 260A is then extended as shown in Figure 25E and ablation is performed to ablate a second tissue segment 275B as shown in Figure 25F. The distal tip 260A becomes the new anchor. The process can be repeated again as needed. The rotation and anchoring steps can be performed by the user with a trigger mechanism that mechanically transmits movement to cause the desired circular movement such that each pull of the trigger will move the device one complete "step," without the user needing to attend to the stages described above. The resulting tool handle held by the performing physician will look and feel like many other endoscopic surgical tools the physician may be used to.

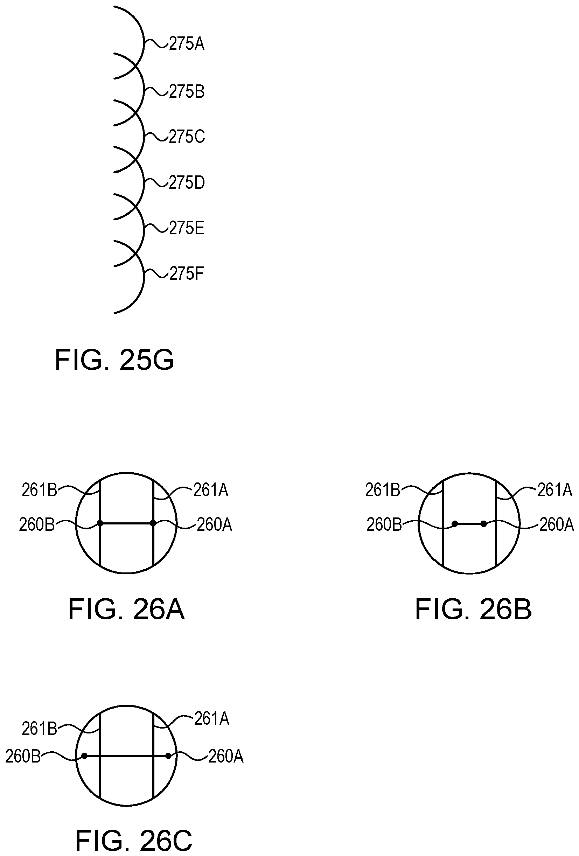

In some embodiments, the area of the tip 260A, 260B has a larger surface area or special coating so that the ablation around this area is less than the ablation along the electrode wire 260C. This area can be ablated at reduced intensity; however, since this zone is actually ablated twice while the ablation line is being drawn, the result is an even ablation along the entire ablation line. Alternatively, no ablation is applied at the "anchoring" zones, and the continuity of the ablation line is achieved by overlapping of a curved foot print as shown in Figure 25G showing overlapping, semi-circular ablation zones 275A, 275B, 275C, 275D, 275E, and 275F. In some embodiments, the electrode 132 coming in contact with the bladder wall can be pre-shaped to have a curved foot print on the bladder wall. For these embodiments, a continuous ablation line can be achieved even if ablations are not carefully continuous, for example, by creating an overlap between adjacent ablation segments.

In some embodiments, the first part of the electrode, i.e., the part extending from the distal exit of the scope 250 to the first anchor, is built to have a specifically predetermined flexibility so that when force is applied to it by pressing the tool 200b against the bladder wall BW, the tool 200b will bend in an expected manner. In other embodiments, other elastic members have the same property-change of length or protrusion of the tool 200B from the scope 250, as a result of the force applied by the tool to the bladder wall BW. This can be achieved by a coiled spring. In some embodiments, the elastic member or coiled spring is sheathed within the scope 250.

In these and other embodiments, when the tool 200b is not pushed against the bladder wall, the tool extends out of the scope 250 to a fixed distance. Thus, the actual distance of the scope 250 from the bladder wall BW depends on deformation of the elastic member and is proportional to the force applied against the bladder wall BW. In some embodiments, the distance of the scope 250 from the bladder wall when the tool 200B is extended and no pressure is applied is set so that a user will immediately visually recognize when enough pressure is applied by visual cues. Such cues may be based on the correct distance of the scope 250 from the wall BW, which is a surrogate of the correct applied pressure. In some embodiments, the optical clue used is the perceived distance between electrode tips 260A, 260B. The desired perceived distance can be set so that the tips 260A, 260B will extend between two markings 261A, 261B within the visual field seen through the scope 250. If correct pressure is applied, the user will see the tips 260A, 260B extend exactly between the markings 261A, 261B as shown in Figure 26A. If insufficient pressure is applied, the user will recognize that the tips 260A, 260B do not span the gap between the markings 261A, 261B as shown in Figure 26B. When too much pressure is applied, the scope will move closer to the bladder wall BW and the distance between the tips 260A, 260B will appear wider than the gap between the markings 261A, 261B as shown in Figure 26C. In some embodiments, the visual field borders are used instead of markings.

Thus, the tool 200B extends out of the scope 250 to a known distance, as long as the tool is not pressed against the bladder wall BW. Once pressed, this length is changed so that when the scope is too near to the bladder wall BW, the user can conclude that the arch or other elastic member is excessively deformed, meaning the force applied is too high to be safe. When the pressure of the electrode tips 260A, 260B against the bladder wall BW exceeds a certain value, ablation may be hazardous, risking bladder wall perforation. If, on the other hand, the user recognizes that the distance between the scope and the bladder wall is too large, the user can conclude that the arch or other elastic member is insufficiently deformed, meaning the electrode is not pressed hard enough against the bladder wall BW, risking ineffective ablation or the formation of coagulum.

In some embodiments, the electrodes of electrode tips 260A, 260B are somewhat arched so that the known elasticity of the arch and two opposing arms of the tool which form "forceps" can be used to maintain the electrode contact with the tissue at a relatively constant value. If the tool 200B is pressed against the bladder with force, the arched electrodes will become flat while forcing the tool "forceps" to open apart. The ablation may be performed only when the distance between the forceps arms is around a certain value, and ablation is aborted when this value is exceeded. In some embodiments, the ablation is not performed unless the distance between the arms of the device 200B exceeds a certain threshold, to avoid ablation when contact with the tissue is poor.

In some embodiments, the desired value of force of the electrode against the bladder tissue is set to be such that the contact pressure will be between 0.01 bar to 0.05 bar, such as 0.02 bar ( 100 grams per cm² to 500 grams per cm², such as 200 grams per cm²).

In some embodiments, the bladder is filled by air or fluid at high pressure during or immediately after the ablations are performed, to avoid contraction of the bladder and actually facilitate augmentation of the bladder volume by stretching the bladder wall, especially at the recently ablated segments or during ablation. Exemplary values will be to inflate the bladder to a pressure of approximately 0.08 bar ( 80 centimeters H₂O) and keep it full for one to five minutes before letting the fluid out.

In some embodiments, the gauging of pressure applied by the electrode is achieved by a pressure sensor. In some embodiments, the extension of the tool out of the scope is determined by the pressure against the bladder wall. In some embodiments, the device is set to automatically limit ablation only to times when the pressure is within a pre-set range around the desired pressure.

In some embodiments, the part of the tool 200b that is used to deliver the RF current or other current or energy to the bladder wall is somewhat curved to fit the curvature of the bladder wall. In some embodiments, this curvature is fitted for a sphere with a radius of between 10 cm to 40 cm.

Overactive bladders, especially those which are overactive due to neurogenic causes or severe obstruction, may be characterized by significant trabeculations caused by detrusor muscle hypertrophy. Hypertrophied muscle bundles create bulges and indentations in the bladder wall, making the inner surface of the bladder extremely irregular. Ablating lines in such a situation may in some cases be difficult as the irregular surface tends to distort the lines and the variable thickness of the bladder wall can require different degrees of energy at different areas.

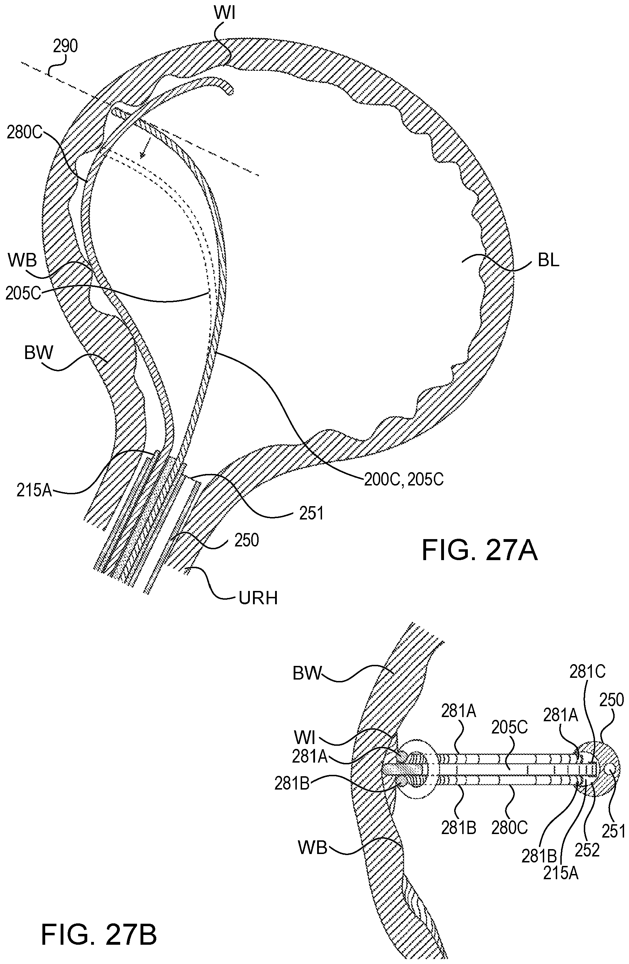

Examples not according to the the invention therefore also provide devices which address the aforementioned issues. Figure 27A shows an ablation tool 200C similar to tool 200 described above. The ablation tool 200C has arced elongate working tip 205C that is extendable out of the shaft 215A into the bladder BL which has bulges WB and indentations WI along its inner surface. The ablation tool 200C further comprises an arced guide 280C preshaped with an arc with a direction opposite that of the working end of the ablation tool 200C. Figure 27B shows a closer top view of the ablation tool 200C. As shown in Figure 27B, the guide 280C has two arms 281A and 281B which are either insulated or made of a non-conductive material.

Figure 27A is a cross-sectional view of the ablation tool 200C in the bladder BL in the coronal plane. Figure 27B is a top view of the ablation tool 200c through the line 290 in Figure 27A. A cystoscope 250 is shown entering the bladder BL through the urethra URH. The cystoscope 250 has optics and a working channel 252 through which the ablation tool 200C, including the shaft 215A, is advanced through. The shaft 215A of the ablation tool 200C includes two rounded lumens 282A, 282B for the two guide arms 281A, 281B and an oblong lumen 281C for the working tip 205C of the ablation tool 200C. The oblong shape of the oblong lumen 281C keeps the working tip 205C and the guide arms 281A, 281B in the same orientation relative to each other. The working tip 205C is held between the two guide arms 281A, 281B. The working tip 205C may further be wider near its distal ends to prevent slipping from between the guide arms 281A, 281B. The ablation tool 200C is shown in two positions in Figure 27A, the first over an indentation WI in the bladder wall as shown with the tip 205C in dotted line and the other over a bulge WB in the bladder wall.

In use, the tool 200C with the guide 280C and the elongate tip 205C retracted in the shaft 215A is inserted through the working channel 252 of the scope 250. Then, the guide 280C and the elongate tip 205C are deployed. The guide 280C then extends from the scope 250 until it touches the bladder wall. The elongate tip 205C is initially at the distal end of the guide 280C and is then gradually drawn towards the scope 250 while ablating. As the elongate tip 205C moves along the inner wall of the bladder, the guide arms 281A, 281B prevent the tool from "skewing" sideways with the ridges the hypertrophied muscles bundles creating the irregular surface.

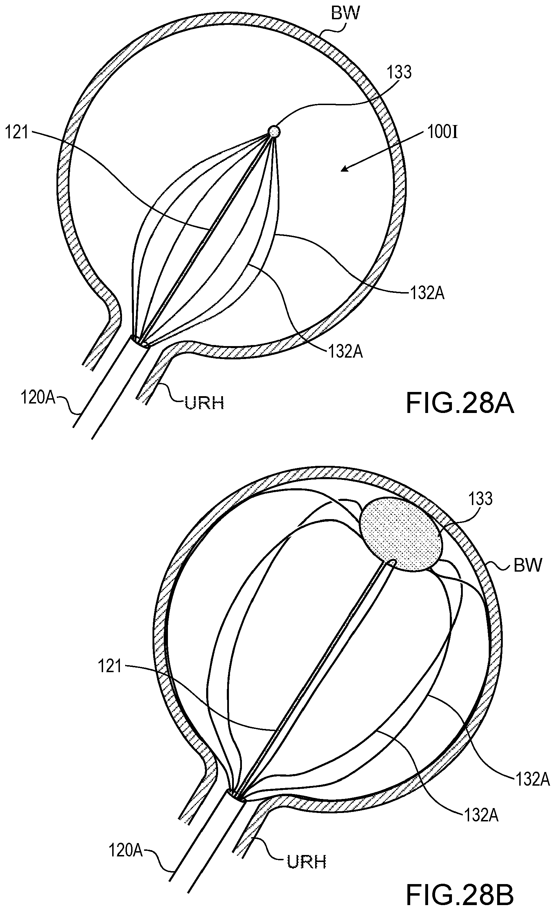

Figures 28A and 28B show another embodiment in which an ablation tool 1001 comprises a cage-like arrangement of struts or electrodes 132A positioned over an inner shaft 121 and has an inflatable balloon 133 at the distal tip of the inner shaft 121. The inner shaft 121 is advancable and retractable within the outer shaft 120A. As the ablation tool 1001 is advanced through the urethra URH, the inflatable balloon 133 can prevent the distal tips of the cage-like arrangement of struts or electrodes 132A from causing injury or perforation of the bladder. The ablation tool 1001 can also be advanced so that the inflatable balloon 133 contacts the bladder dome. The contact between the inflatable balloon 133 and the bladder dome can facilitate finding the right depth of insertion of the device. Also, the balloon 133 can act as an anchor. Further, the balloon can provide a counter force to the struts 132A while they are being pushed into the bladder and can help them better orient in the appropriate curve aligned with the bladder curvature.

Figure 28A is a side view of the bladder and the ablation tool 1001 before the inflatable balloon 133I is inflated. The ablation tool 100i is shown in Figure 28A as partially inserted into the bladder. In Figure 28B, the bladder has been inflated, the ablation tool 1001 is advanced until it reaches the dome of the bladder, and the struts 132 are pushed into the bladder so that they make contact with the inner wall of the bladder. The balloon 133 can be inflated through the inner lumen of the inner shaft 121 positioned in the center of the cage-like arrangement of struts 132a. Once positioned against the inner wall of the bladder, the electrodes or struts 132 can be energized to create a pattern of longitudinal ablation lines as shown, for example, in Figure 13.

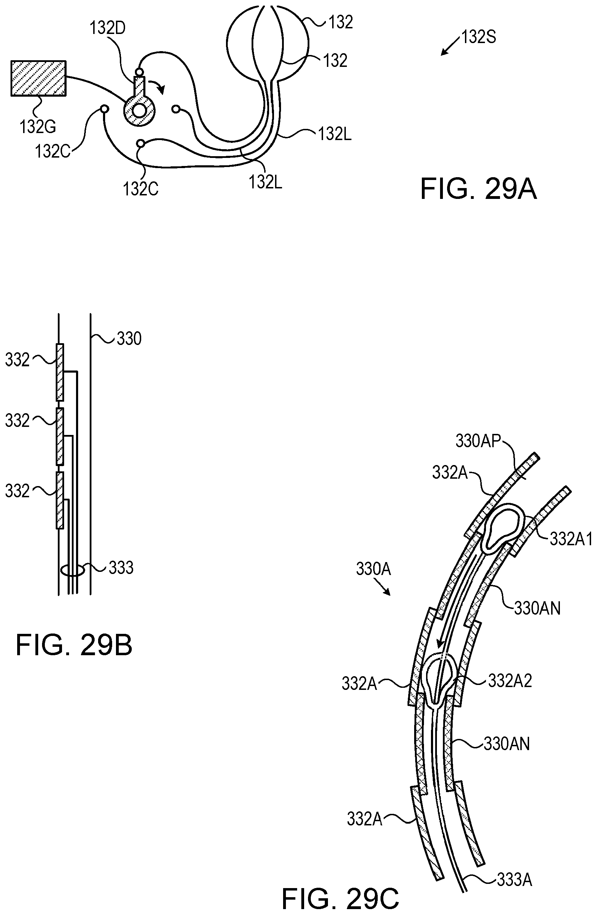

In many embodiments, an ablation tool having multiple electrodes may alternate ablation between different electrode segments. Figure 29A is a schematic drawing of an electrical system 132S which can be used with many of the ablation tools described herein such as ablation tool 100. The system 132S comprises a plurality of electrodes 132 powered through a plurality of electrode leads 132L ending at contact points 132C. The system 132S comprises a mechanical distributor 132 that is used to alternate the ablation energy between the electrodes 132. A power generator 132G is connected to a mechanical distributor arm 132D which rotates and touches different electrode contact points 132C, delivering energy to individual electrodes 132 at different moments. The speed of rotation and the width of the arm or contacts of the distributor 132D can be altered to control the timing of energy delivery. The mechanical distributor arm 132D can be rotated by an electric motor. In other embodiments, a non-mechanical electrical system using electronic switches may be used to alternate energy delivery amongst individual electrodes 132.

Figure 29B is a schematic representation of a strut 330 which can be used with many of the ablation tools described herein such as ablation tool 100. For example, one or more of the struts 330 can be disposed over the balloon 110 of the ablation tool 100 to contact the inner wall of the hollow organ. The strut 330 is attached to separate conductive areas 332 acting as ablation electrodes. Each such electrode 332 is connected via an insulated wire 333 to an electrical distributor such as the mechanical distributor arm 132D discussed above. Thus, ablation energy can be alternated between conductive areas 332.

Figure 29C is a schematic representation of another strut 330A which can be used with many of the ablation tools described herein such as ablation tool 100. For example, one or more of the struts 330 can be disposed over the balloon 110 of the ablation tool 100 to contact the inner wall of the hollow organ. The strut 330A can comprise a wire 333A running through a passageway 330AP inside of the strut 330A. The wire 333A comprises insulated segments and non-insulated contact points having a flexible widening at its distal end. The strut 330A comprises conductive segments 332A and non-conductive segments 330AN. The non-conductive segments 330AN are slightly narrower than the conductive segments 332A. The wire 333A may be pulled through the passageway 330AP of the strut 330A and due to the shape and width of the passageway 330AP will get temporarily stuck at the distal end of each non-conductive segment 330AN, while remaining in contact with the conductive segment 332A. If pulled more forcefully, the contact will "give" and pass through the narrow segment, until the wire 333A becomes "stuck" again at the next narrowing.

In use, the strut 330A may be provided with the wire 333A contacting at the most distal position in the passageway 330AP. After deployment of a device in the bladder having one or more struts 330A disposed thereon, ablation is performed at the first conductive segment 332A1. The user then pulls the wire outwards until the stop at the next segment 332A2 to conduct ablation. The process is repeated for all segments 332A.

While preferred embodiments of the present invention have been shown and described herein, it will be obvious to those skilled in the art that such embodiments are provided by way of example only. Numerous variations, changes, and substitutions will now occur to those skilled in the art . It should be understood that various alternatives to the embodiments described herein may be employed in practicing the invention. It is intended that the following claims define the scope of the invention .

## Claims

1. A device for treating a urinary disorder in a urinary bladder, the device comprising:
a shaft (120; 120A; 120B) advancable through a urethra of a patient to reach the bladder;
a tissue modification structure (110; 110A; 110B) coupled to a distal end of the shaft, wherein at least a portion of the tissue modification structure is adapted to contact and conform to at least a portion of an inner wall of the bladder; and
means (132; 133) for creating a predetermined pattern of tissue regions having reduced electrical propagation in the inner wall of the bladder to modify at least one of a mechanical or an electrical property of the bladder,
wherein the means (132; 133) for creating the predetermined pattern of tissue regions comprises a plurality of spaced apart conductors (132) configured to generate an ablation line, the ablation line being continuous only if the bladder volume is low and defining the predetermined pattern of tissue regions having reduced electrical propagation.

2. The device of claim 1, wherein the means (132; 133) for creating the predetermined pattern of tissue regions is configured to apply energy to the plurality of conductors (132) to cause the tissue regions to extend through the urothelium, through the urothelium and the suburothelium, the urothelium and suburothelium and at least a part of the detrusor, or through an entire wall of the bladder.

3. The device of claim 1, wherein the tissue modification structure (110; 110A; 110B) comprises a cage shaped to conform to the inner wall of the bladder, said cage carrying the plurality of conductors (132).

4. The device of claim 3, wherein the cage comprises one or more mapping electrodes configured to locate foci of aberrant electrical activity in the bladder, optionally wherein the predetermined pattern of tissue regions having reduced electrical propagation corresponds to the located foci of aberrant electrical activity in the bladder.

5. The device of claim 1, wherein the plurality of conductors (132) is adapted to create the predetermined pattern of tissue regions having reduced electrical propagation by at least one of RF ablation, cryoablation, photoablation, microwave energy, use of a chemical agent, ultrasound energy, and mechanical scoring.

6. The device of claim 3, wherein the cage is expandable within the bladder to move the plurality of conductors (132) to create the predetermined pattern.

7. The device of claim 1, wherein the plurality of conductors is adapted to deliver energy to the inner wall of the bladder selectively or simultaneously.

8. The device of claim 1, 2, or 3, wherein the tissue modification structure (110; 110A; 110B) comprises an elongate curved element shaped to conform to at least a portion of the inner wall of the bladder, and optionally wherein the elongate curved element is electrically coupled to a power generator at a plurality of points along the elongate curved element.

9. The device of claim 8, wherein the plurality of conductors (132) comprises one or more active elements disposed on the elongate curved element.

10. The device of claim 9, wherein the plurality of conductors (132) is adapted to create the predetermined pattern of tissue regions having reduced electrical propagation by at least one of RF ablation, cryoablation, photoablation, microwave energy, use of a chemical agent, ultrasound energy, and mechanical scoring, or wherein the elongate curved element (110; 110A; 110B) is expandable to move the plurality of conductors (132) to create the predetermined pattern.

11. The device of claim 1, 2, or 3, further comprising an expandable element coupled with the tissue modification structure (110; 110A; 110B) and expandable to press the tissue modification structure against the inner wall of the bladder.

12. The device of any preceding claim, wherein the at least one conductor (132) is configured to generate lines having a width in a range from 1 mm to 10 mm, lines separated by a distance in a range from 10 mm to 150 mm.

13. The device of claim 12, wherein the means (132; 133) for creating the plurality of ablation lines is configured to apply energy to the plurality of conductors (132) to create the plurality of ablation lines to minimise contact between the ablation lines when the bladder is collapsed or to avoid one or more anatomical regions selected from a group comprising the ureteral orifice, the uretero vesical junction, the trigone area, the bladder dome, or the bladder outlet.

14. The device of any preceding claim, wherein the means (132; 133) for creating the predetermined pattern of tissue regions is configured to apply energy to the plurality of conductors (132) to electrically isolate one or more anatomical regions in the bladder.

## Patentansprüche

1. Vorrichtung zur Behandlung einer Harnwegserkrankung in einer Harnblase, wobei die Vorrichtung umfasst:
einen Schaft (120; 120A; 120B), der durch die Harnröhre eines Patienten vorschiebbar ist, um die Blase zu erreichen;
eine Gewebemodifikationsstruktur (110; 110A; 110B), die mit einem distalen Ende des Schafts verbunden ist, wobei mindestens ein Teil der Gewebemodifikationsstruktur so angepasst ist, dass sie mindestens einen Teil einer Innenwand der Blase berührt und sich an diese anpasst; und
Mittel (132; 133) zur Erzeugung eines vorbestimmten Musters von Gewebebereichen mit reduzierter elektrischer Ausbreitung in der Innenwand der Blase, um mindestens eine mechanische oder eine elektrische Eigenschaft der Blase zu verändern,
wobei die Mittel (132; 133) zum Erzeugen des vorbestimmten Musters von Gewebebereichen eine Vielzahl von voneinander beabstandeten Leitern (132) umfassen, die so konfiguriert sind, dass sie eine Ablationslinie erzeugen, wobei die Ablationslinie nur dann durchgehend ist, wenn das Blasenvolumen gering ist und das vorbestimmte Muster von Gewebebereichen mit reduzierter elektrischer Ausbreitung definiert.

2. Vorrichtung nach Anspruch 1, wobei die Mittel (132; 133) zum Erzeugen des vorbestimmten Musters von Gewebebereichen so konfiguriert sind, dass sie Energie an die mehreren Leiter (132) anlegen, um zu bewirken, dass sich die Gewebebereiche durch das Urothelium, durch das Urothelium und das Suburothelium, das Urothelium und das Suburothelium und mindestens einen Teil des Detrusors oder durch eine gesamte Blasenwand erstrecken.

3. Vorrichtung nach Anspruch 1, wobei die Gewebemodifikationsstruktur (110; 110A; 110B) einen Käfig umfasst, der so geformt ist, dass er sich an die Innenwand der Blase anpasst, wobei der Käfig die Vielzahl von Leitern (132) trägt.

4. Vorrichtung nach Anspruch 3, wobei der Käfig eine oder mehrere Kartierungselektroden umfasst, die so konfiguriert sind, dass sie Herde abweichender elektrischer Aktivität in der Blase lokalisieren, wobei das vorbestimmte Muster von Gewebebereichen mit reduzierter elektrischer Ausbreitung optional den lokalisierten Herden abweichender elektrischer Aktivität in der Blase entspricht.

5. Vorrichtung nach Anspruch 1, bei der die Vielzahl von Leitern (132) so beschaffen ist, dass sie das vorbestimmte Muster von Gewebebereichen mit reduzierter elektrischer Ausbreitung durch mindestens eines der folgenden Verfahren erzeugt: HF-Ablation, Kryoablation, Photoablation, Mikrowellenenergie, Verwendung eines chemischen Mittels, Ultraschallenergie und mechanische Ritzung.

6. Vorrichtung nach Anspruch 3, wobei der Käfig innerhalb der Blase ausdehnbar ist, um die Vielzahl von Leitern (132) zu bewegen, um das vorbestimmte Muster zu erzeugen.

7. Vorrichtung nach Anspruch 1, bei der die Vielzahl von Leitern so ausgelegt ist, dass sie selektiv oder gleichzeitig Energie an die Innenwand der Blase abgeben.

8. Vorrichtung nach Anspruch 1, 2 oder 3, wobei die Gewebemodifikationsstruktur (110; 110A; 110B) ein längliches, gekrümmtes Element umfasst, das so geformt ist, dass es sich an mindestens einen Teil der Innenwand der Blase anpasst, und wobei das längliche, gekrümmte Element optional an mehreren Punkten entlang des länglichen, gekrümmten Elements elektrisch mit einem Stromgenerator verbunden ist.

9. Vorrichtung nach Anspruch 8, wobei die Vielzahl von Leitern (132) ein oder mehrere aktive Elemente umfasst, die auf dem länglichen gebogenen Element angeordnet sind.

10. Vorrichtung nach Anspruch 9, wobei die Vielzahl von Leitern (132) so angepasst ist, dass sie das vorbestimmte Muster von Gewebebereichen mit reduzierter elektrischer Ausbreitung durch mindestens eines der folgenden Verfahren erzeugt: HF-Ablation, Kryoablation, Photoablation, Mikrowellenenergie, Verwendung eines chemischen Mittels, Ultraschallenergie und mechanisches Einritzen, oder wobei das längliche gekrümmte Element (110; 110A; 110B) expandierbar ist, um die Vielzahl von Leitern (132) zu bewegen, um das vorbestimmte Muster zu erzeugen.

11. Vorrichtung nach Anspruch 1, 2 oder 3, umfassend ferner ein ausdehnbares Element, das mit der Gewebemodifikationsstruktur (110; 110A; 110B) verbunden und ausdehnbar ist, um die Gewebemodifikationsstruktur gegen die Innenwand der Blase zu drücken.

12. Vorrichtung nach einem der vorhergehenden Ansprüche, wobei der mindestens eine Leiter (132) so konfiguriert ist, dass er Linien mit einer Breite in einem Bereich von 1 mm bis 10 mm erzeugt, wobei die Linien durch einen Abstand in einem Bereich von 10 mm bis 150 mm getrennt sind.

13. Vorrichtung nach Anspruch 12, wobei das Mittel (132; 133) zum Erzeugen der Vielzahl von Ablationslinien so konfiguriert ist, dass es Energie auf die Vielzahl von Leitern (132) anwendet, um die Vielzahl von Ablationslinien zu erzeugen, um den Kontakt zwischen den Ablationslinien zu minimieren, wenn die Blase kollabiert ist, oder um einen oder mehrere anatomische Bereiche zu vermeiden, die aus einer Gruppe ausgewählt sind, die die Harnleiteröffnung, die Uretero-Vesikus-Verbindung, den Trigonusbereich, die Blasenkuppel oder den Blasenauslass umfasst.

14. Vorrichtung nach einem der vorhergehenden Ansprüche, wobei die Mittel (132; 133) zum Erzeugen des vorbestimmten Musters von Gewebebereichen so konfiguriert sind, dass sie Energie auf die Vielzahl von Leitern (132) aufbringen, um eine oder mehrere anatomische Bereiche in der Blase elektrisch zu isolieren.

## Revendications

1. Dispositif destiné à traiter un trouble urinaire dans une vessie urinaire, le dispositif comprenant :
un arbre (120 ; 120A ; 120B) capable d'avancer à travers un urètre d'un patient pour atteindre la vessie ;
une structure de modification de tissu (110 ; 110A ; 110B) couplée à une extrémité distale de l'arbre, dans lequel au moins une partie de la structure de modification de tissu est conçue pour entrer en contact avec au moins une partie d'une paroi interne de la vessie et épouser la forme de cette partie ; et
un moyen (132 ; 133) destiné à créer un motif prédéterminé de régions de tissu ayant une propagation électrique réduite dans la paroi interne de la vessie pour modifier au moins une propriété parmi une propriété mécanique ou électrique de la vessie,
dans lequel le moyen (132 ; 133) destiné à créer le motif prédéterminé de régions de tissu comprend une pluralité de conducteurs (132) espacés les uns des autres configurés pour générer une ligne d'ablation, la ligne d'ablation étant continue uniquement si le volume de la vessie est bas et définissant le motif prédéterminé de régions de tissu ayant une propagation électrique réduite.

2. Dispositif selon la revendication 1, dans lequel le moyen (132 ; 133) destiné à créer le motif prédéterminé de régions de tissu est configuré pour appliquer de l'énergie à la pluralité de conducteurs (132) pour amener les régions de tissu à s'étendre à travers l'urothélium, à travers l'urothélium et le suburothélium, l'urothélium et le suburothélium et au moins une partie du détrusor, ou à travers une paroi entière de la vessie.

3. Dispositif selon la revendication 1, dans lequel la structure de modification de tissu (110 ; 110A ; 110B) comprend une cage façonnée pour épouser la forme de la paroi interne de la vessie, ladite cage portant la pluralité de conducteurs (132).

4. Dispositif selon la revendication 3, dans lequel la cage comprend une ou plusieurs électrodes de mappage configurées pour localiser des foyers d'activité électrique anormale dans la vessie, facultativement dans lequel le motif prédéterminé de régions de tissu ayant une propagation électrique réduite correspond aux foyers localisés d'activité électrique anormale dans la vessie.

5. Dispositif selon la revendication 1, dans lequel la pluralité de conducteurs (132) est conçue pour créer le motif prédéterminé de régions de tissu ayant une propagation électrique réduite par au moins un élément parmi une ablation par RF, une cryoablation, une photoablation, une énergie micro-onde, l'utilisation d'un agent chimique, une énergie ultrasonore, et une incision mécanique.

6. Dispositif selon la revendication 3, dans lequel la cage est capable d'expansion au sein de la vessie pour déplacer la pluralité de conducteurs (132) pour créer le motif prédéterminé.

7. Dispositif selon la revendication 1, dans lequel la pluralité de conducteurs est conçue pour délivrer de l'énergie à la paroi interne de la vessie sélectivement ou simultanément.

8. Dispositif selon la revendication 1, 2, ou 3, dans lequel la structure de modification de tissu (110 ; 110A ; 110B) comprend un élément courbé allongé façonné pour épouser la forme d'au moins une partie de la paroi interne de la vessie, et facultativement dans lequel l'élément courbé allongé est couplé électriquement à un générateur de puissance au niveau d'une pluralité de points le long de l'élément courbé allongé.

9. Dispositif selon la revendication 8, dans lequel la pluralité de conducteurs (132) comprend un ou plusieurs éléments actifs disposés sur l'élément courbé allongé.

10. Dispositif selon la revendication 9, dans lequel la pluralité de conducteurs (132) est conçue pour créer le motif prédéterminé de régions de tissu ayant une propagation électrique réduite par au moins un élément parmi une ablation par RF, une cryoablation, une photoablation, une énergie micro-onde, l'utilisation d'un agent chimique, une énergie ultrasonore, et une incision mécanique, ou dans lequel l'élément courbé allongé (110 ; 110A ; 110B) est capable d'expansion pour déplacer la pluralité de conducteurs (132) pour créer le motif prédéterminé.

11. Dispositif selon la revendication 1, 2, ou 3, comprenant en outre un élément capable d'expansion couplé à la structure de modification de tissu (110 ; 110A ; 110B) et capable d'expansion pour appuyer la structure de modification de tissu contre la paroi interne de la vessie.

12. Dispositif selon l'une quelconque des revendications précédentes, dans lequel l'au moins un conducteur (132) est configuré pour générer des lignes ayant une largeur comprise dans une plage allant de 1 mm à 10 mm, des lignes séparées par une distance comprise dans une plage allant de 10 mm à 150 mm.

13. Dispositif selon la revendication 12, dans lequel le moyen (132 ; 133) destiné à créer la pluralité de lignes d'ablation est configuré pour appliquer de l'énergie à la pluralité de conducteurs (132) pour créer la pluralité de lignes d'ablation pour minimiser le contact entre les lignes d'ablation quand la vessie est rétractée ou pour éviter une ou plusieurs régions anatomiques choisies parmi un groupe comprenant l'orifice urétéral, la jonction urétéro-vésicale, la zone du trigone, le dôme vésical, ou l'orifice de la vessie.

14. Dispositif selon l'une quelconque des revendications précédentes, dans lequel le moyen (132 ; 133) destiné à créer le motif prédéterminé de régions de tissu est configuré pour appliquer de l'énergie à la pluralité de conducteurs (132) pour isoler électriquement une ou plusieurs régions anatomiques dans la vessie.
